# EUROPEAN PATENT SPECIFICATION

(11) **EP 2 414 521 B1**
(45) Date of publication and mention of the grant of the patent: **26.10.2016**
(21) Application number: 10764855.2
(22) Date of filing: 31.03.2010
(51) Int. Cl.: C12N 15/113, C12N 15/85, A61K 31/7105, A61P 3/06

(54) **REGULATION OF MIR-33 MICRORNAS IN THE TREATMENT OF CHOLESTEROL-RELATED DISORDERS**
REGULIERUNG VON MIR-33-MIKRORNAS BEI DER BEHANDLUNG CHOLESTERINSPIEGELBEDINGTER ERKRANKUNGEN
RÉGULATION DES MICROARN MIR-33 DANS LE TRAITEMENT DES TROUBLES LIÉS AU CHOLESTÉROL

(30) Priority: 31.03.2009 US 165041 P
(43) Date of publication of application: 08.02.2012
(73) Proprietor: The General Hospital Corporation, Boston, MA 02114 (US)
(72) Inventor: NAAR, Anders M., Arlington Massachusetts 02474 (US)
(74) Representative: Sutcliffe, Nicholas Robert
(86) International application number: PCT/US2010/029376
(87) International publication number: WO 2010/120508

(56) References cited:
- WO-A2-2008/061537
- US-A1- 2005 261 319
- US-A1- 2008 206 232
- HARRY R. DAVIS, JR. ET AL.: 'Niemann-Pick C1 Like 1 (NPC1L1) Is the Intestinal Phytosterol and Cholesterol Transporter and a Key Modulator of Whole-body Cholesterol Homeostasis.' THE JOURNAL OF BIOLOGICAL CHEMISTRY vol. 279, no. 32, 2004, pages 33586 - 33592, XP002495332
- GEORGE E. 0. MUSCAT ET AL.: 'Regulation of Cholesterol Homeostasis and Lipid Metabolism in Skeletal Muscle by Liver X Receptors.' THE JOURNAL OF BIOLOGICAL CHEMISTRY vol. 277, no. 43, 2002, pages 40722 - 40728, XP002981432
- PABLO LANDGRAF ET AL.: 'A Mammalian microRNA Expression Atlas Based on Small RNA Library Sequencing.' CELL. vol. 129, no. 7, 2007, pages 1401 - 1414, XP002490508
- NOAM ZELCER ET AL.: 'Attenuation of neuroinflammation and Alzheimer's disease pathology by liver x receptors.' PNAS. vol. 104, no. 25, 2007, pages 10601 - 10606, XP008167626
- SAM GRIFFITHS-JONES ET AL.: 'miRBase: microRNA sequences, targets and gene nomenclature.' NUCLEIC ACIDS RESEARCH vol. 34, no. 1, 2006, pages D140 - D144, XP009108075
- DATABASE GENBANK 29 October 2007 'Homo sapiens microRNA mir-33b', XP008167627 Database accession no. AJ550398
- DATABASE GENBANK 11 June 2003 'Homo sapiens microRNA miR-33', XP008167637 Database accession no. AJ421755

### SEQUENCE LISTING

<110> The General Hospital Corporation
<120> Use of MIR-33 MicroRNAs in the Treatment of Cholesterol-Related
   Disorders
<130> 00786-0686WO1
<150> US 61/165,041
   <151> 2009-03-31
<160> 20
<170> FastSEQ for Windows Version 4.0
<210> 1
   <211> 21
   <212> RNA
   <213> Human miR-33 a
<400> 1
   gugcauugua guugcauugc a 21
<210> 2
   <211> 20
   <212> RNA
   <213> Human miR-33 b
<400> 2
   gugcauugcu guugcauugc 20
<210> 3
   <211> 21
   <212> RNA
   <213> Artificial Sequence
<220>
   <223> Anti-miR-33a oligonucleotide
<400> 3
   ugcaaugcaa cuacaaugca c 21
<210> 4
   <211> 20
   <212> RNA
   <213> Artificial Sequence
<220>
   <223> Anti-miR-33b oligonucleotide
<400> 4
   gcaaugcaac agcaaugcac 20
<210> 5
   <211> 39
   <212> DNA
   <213> Artificial Sequence
<220>
   <223> Fragment of human ABCA1 gene
<400> 5
   tattcaatgc aatgcaattc aatgcaatga aaacaaaat 39
<210> 6
   <211> 19
   <212> RNA
   <213> Artificial Sequence
<220>
   <223> Antisense to Human Micro RNA 33a
<400> 6
   caaugcaacu acaaugcac 19
<210> 7
   <211> 19
   <212> RNA
   <213> Human Micro RNA 33a
<400> 7
   gugcauugua guugcauug 19
<210> 8
   <211> 21
   <212> RNA
   <213> Artificial Sequence
<220>
   <223> Antisense to Human Micro RNA 33b
<400> 8
   ugcaaugcaa cagcaaugcu c 21
<210> 9
   <211> 21
   <212> RNA
   <213> Human Micro RNA 33b
<400> 9
   gugcauugcu guugcauugc a 21
<210> 10
   <211> 19
   <212> RNA
   <213> Artificial Sequence
<220>
   <223> Mutant form of Human Micro RNA 33a
<400> 10
   guuccuugua guugcauug 19
<210> 11
   <211> 21
   <212> RNA
   <213> Artificial Sequence
<220>
   <223> Mutant form of Human Micro RNA 33b
<400> 11
   guuccuugcu guugcauugc a 21
<210> 12
   <211> 96
   <212> DNA
   <213> Artificial Sequence
<220>
   <223> Fragment of human SREBP-1 gene
<400> 12
   gcgggcggcc ccgcggtgca ttgctgttgc attgcacgtg tgtgaggcgg gtgcagtgcc 60
   tcggcagtgc agcccggagc cggcccctgg caccac 96
<210> 13
   <211> 69
   <212> DNA
   <213> Artificial Sequence
<220>
   <223> Fragment of human SREBP-2 gene
<400> 13
   ctgtggtgca ttgtagttgc attgcatgtt ctggtggtac ccatgcaatg tttccacagt 60
   gcatcacag                                                          69
<210> 14
   <211> 69
   <212> RNA
   <213> Artificial Sequence
<220>
   <223> Fragment of human SREBF-2 gene
<400> 14
   cuguggugca uuguaguugc auugcauguu cuggugguac ccaugcaaug uuuccacagu 60
   gcaucacag                                                          69
<210> 15
   <211> 69
   <212> RNA
   <213> Artificial Sequence
<220>
   <223> Fragment of mouse SREBF-2 gene
<400> 15
   cuguggugca uuguaguugc auugcauguu cuggcaauac cugugcaaug uuuccacagu 60
   gcaucacgg                                                          69
<210> 16
   <211> 69
   <212> RNA
   <213> Artificial Sequence
<220>
   <223> Fragment of chicken SREBF-2 gene
<400> 16
   cuguagugca uuguaguugc auugcaugug cuggcaguaa cugugcaaug uuccugcagu 60
   gcaguacag                                                          69
<210> 17
   <211> 68
   <212> RNA
   <213> Artificial Sequence
<220>
   <223> Fragment of putative frog SREBF-1 gene
<400> 17
   cuguggugca uuguaguugc auugcaugug auaucagcgg ugugcaaugu gccugcagug 60
   caacacag                                                          68
<210> 18
   <211> 99
   <212> RNA
   <213> Artificial Sequence
<220>
   <223> Fragment of D. Melanogaster SREBF-2 gene
<400> 18
   cucuuccucu ggagaugaca cgaaggugca uuguagucgc auugucuguc ccaauugcuu 60
   caggcaauac aacuucagug caagcucugu gcauuucac                        99
<210> 19
   <211> 96
   <212> RNA
   <213> Artificial Sequence
<220>
   <223> Fragment of human SREBF-1 gene
<400> 19
   gcgggcggcc ccgcggugca uugcuguugc auugcacgug ugugaggcgg gugcagugcc 60
   ucggcagugc agcccggagc cggccccugg caccac                           96
<210> 20
   <211> 69
   <212> RNA
   <213> Artificial Sequence
<220>
   <223> Fragment of frog SREBF-2 gene
<400> 20
   cccuggugca uuguuguugc auugcauguc accuuggacg uugugcaaug uuucuucagu 60
   gcaguaugg                                                         69

## Description

### BACKGROUND OF THE INVENTION

Abnormal cholesterol and lipid homeostasis are linked with prevalent diseases such as metabolic syndrome, atherosclerosis/cardiovascular disease, and type 2 diabetes. Cholesterol and lipids are trafficked in the blood as lipoprotein particles, such as low-density lipoprotein (LDL) and high-density lipoprotein (HDL) that ferry their fatty cargo to different cells and tissues. Excess circulating LDL can be oxidized and taken up by arterial macrophages, turning them into cholesterol/lipid-filled "foam cells" that are involved in the formation of atherosclerotic plaques. Conversely, HDL-cholesterol is exported from peripheral cells, including macrophages, and trafficked to the liver for disposal by the reverse cholesterol transport pathway however, patients suffering from heart disease often have low circulating HDL and decreased cholesterol clearance. Hence, therapeutic efforts to ameliorate cardiovascular disease have centered on lowering cholesterol biosynthesis and LDL production (e.g. by statin treatment), as well as increasing HDL-mediated reverse cholesterol transport (e.g. by niacin supplementation). Although these treatments have shown considerable efficacy, cardiovascular disease remains the leading cause of death in the United States. A detailed understanding of the molecular mechanisms of cholesterol and lipid regulation might facilitate the development of novel therapeutic strategies to treat cardiovascular disease.

WO2008/061537 discloses (cf. SEQ ID No. 298, claims 3, 91) oligonucleotides complementary to position 1-20 of any SEQ ID Nos: 1-723, of which SEQ ID No. 298 corresponds to SEQ ID No. 2 of the present application, for use in the treatment of cancer, viral infection, immunological disease or cardiovascular disease.

The ATP-binding cassette transporter ABCA1 is important for HDL synthesis and reverse cholesterol transport from peripheral tissues, including macrophages/foam cells. Mutations/SNPs in the ABCA1 gene have been implicated in atherosclerosis. A novel therapeutic capable of, for example, increasing ABCA1 levels, HDL production, and/or reverse cholesterol transport could improve regulation and potentially, ameliorate cardiovascular disease.

### SUMMARY OF THE INVENTION

The sterol regulatory element binding protein (SREBP) transcription factors are known regulators of genes involved in both cholesterol and lipid biosynthesis and uptake. The presence of sequences encoding conserved microRNAs that are embedded within intronic sequences in the human SREBP-2 and -1 genes have now been discovered. Preliminary studies described herein below yielded the surprising finding that miR-33 a and b, referred to herein as SEQ ID NOs. 1 and 2, respectively, target the ATP-binding cassette transporter ABCA1 for translational repression. The coordinated role of miR-33 with the SREBP host gene products in the regulation of cholesterol homeostasis in mammals was previously unknown. Moreover, it has now been discovered that inhibition of miR-33 represents a novel therapeutic means to increase ABCA1 levels, HDL production, and reverse cholesterol transport.

In one aspect, the invention provides an isolated nucleic acid sequence that is complementary to SEQ ID NOs. 1 or 2, for use as set out in claim 1. The nucleic acid may be effective to increase the expression of the ATP-binding cassette, subfamily A, member 1, (ABCA1) protein in a mammalian cell. In some embodiments, the composition comprises the nucleic acid in an amount effective to increase the expression of the ATP-binding cassette, subfamily A, member 1, (ABCA1) protein in a human subject in need of cholesterol homeostasis.

In one embodiment, the nucleic acid sequence that is complementary to SEQ ID NOs. 1 or 2 can be an antisense oligonucleotide, including but not limited to the antisense oligonucleotides described herein, e.g., SEQ ID NOs:3, 4, 6, or 8. In some embodiments the nucleic acid is modified, e.g., contains one or more non-natural nucleosides. In some embodiments, the nucleic acid is locked.

In another embodiment, the nucleic acid sequence that is complementary to SEQ ID NOs. 1 or 2 can be an interfering RNA, including but not limited to an shRNA or siRNA.

In yet another embodiment, the nucleic acid sequence that is complementary to SEQ ID NOs. 1 or 2 is an antagomir.

In yet another embodiment, the nucleic acid sequence that is complementary to SEQ ID NO. 1 inhibits post-transcriptional processing of SEQ ID NO. 1.

In yet another embodiment, the nucleic acid sequence that is complementary to SEQ ID NO. 2 inhibits post-transcriptional processing of SEQ ID NO. 2. Described herein is

Described herein is a composition comprising a vector capable of expressing a nucleic acid sequence complementary to SEQ ID NOs. 1 or 2, in an amount effective to increase the expression of the ATP-binding cassette, subfamily A, member 1, (ABCA1) protein in a human subject in need of cholesterol homeostasis.

The vector can be a plasmid vector or a viral vector.

Described herein is a method of increasing the expression of the ATP-binding cassette, subfamily A, member 1, (ABCA1) protein in a cell, e.g., a cell in a subject in need of cholesterol homeostasis, the method comprising administering to the cell or subject a nucleic acid sequence that is complementary to SEQ ID NOs. 1 or 2, thereby increasing the expression of the ABCA1 protein in the cell or subject.

Described herein is a method of increasing efflux of intracellular cholesterol and/or production of HDL in the liver of a subject comprising administering to the subject a nucleic acid sequence that is complementary to SEQ ID NOs. 1 or 2, thereby increasing efflux of intracellular cholesterol and/or production of HDL in the liver of the subject.

Further described herein is a method of decreasing the amount of cholesterol circulating the blood of a subject comprising administering to the subject a nucleic acid sequence that is complementary to SEQ ID NOs. 1 or 2, thereby decreasing the amount of cholesterol circulating the blood of the subject.

Other features and advantages of the invention will be apparent from the detailed description, and from the claims. Thus, other aspects of the invention are described in the following disclosure and are within the ambit of the invention.

### BRIEF DESCRIPTION OF THE DRAWINGS

The following Detailed Description, given by way of example, but not intended to limit the invention to specific embodiments described, may be understood in conjunction with the accompanying figures, incorporated herein by reference.

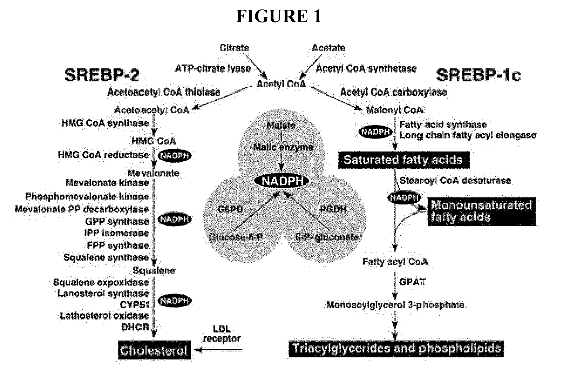

Figure 1 depicts genes regulated by SREBPs. The diagram shows the major metabolic intermediates in the pathways for synthesis of cholesterol, fatty acids, and triglycerides. In vivo, SREBP-2 preferentially activates genes of cholesterol metabolism, whereas SREBP-1c preferentially activates genes of fatty acid and triglyceride metabolism. DHCR, 7-dehydrocholesterol reductase; FPP, farnesyl diphosphate; GPP, geranylgeranyl pyrophosphate synthase; CYP51, lanosterol 14α-demethylase; G6PD, glucose-6-phosphate dehydrogenase; PGDH, 6-phosphogluconate dehydrogenase; GPAT, glycerol-3-phosphate acyltransferase. Adapted from Horton et al. J Clin Invest. 2002. 109:1125-31.

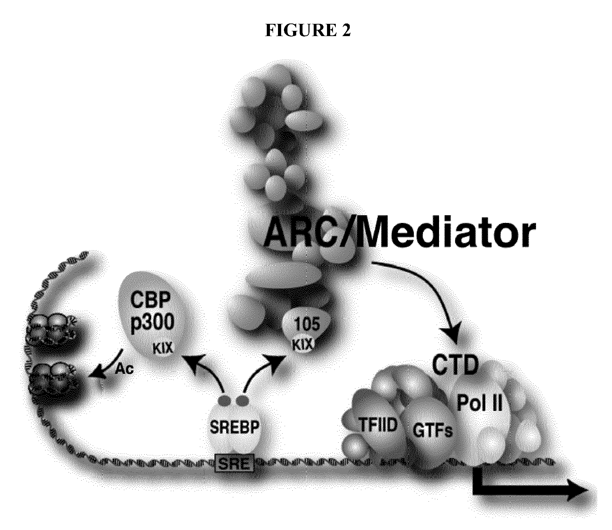

Figure 2 depicts SREBPs activation of transcription of target genes by interacting with structurally conserved KIX domains (blue ovals) present in the CBP/p300 histone acetyltransferases and in the ARC105/MED15 subunit of the ARC/Mediator co-activator complex. Recruitment of CBP/p300 results in increased acetylation of histones/chromatin, whereas ARC/Mediator interaction results in recruitment of RNA Polymerase II (Pol II) via the C-terminal domain (CTD) on its large subunit. TFIID and other general transcription factors (GTFs) are also recruited to the promoter, facilitating initiation of transcription.

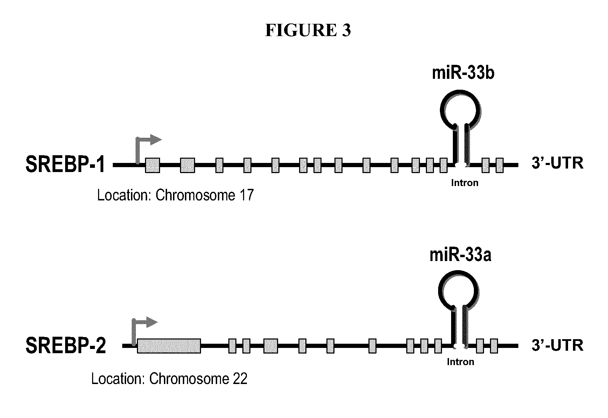

Figure 3 depicts the human SREBP-1 and SREBP-2 genes, which harbor sequences encoding miRNAs (miR-33b and miR-33a, respectively) located in introns. Notably, mice only harbor the miR-33a miRNA in the mouse SREBP-2 gene, whereas the corresponding mouse SREBP-1 intron lacks homology to the human SREBP-1 gene and is devoid of miRNA-encoding sequences.

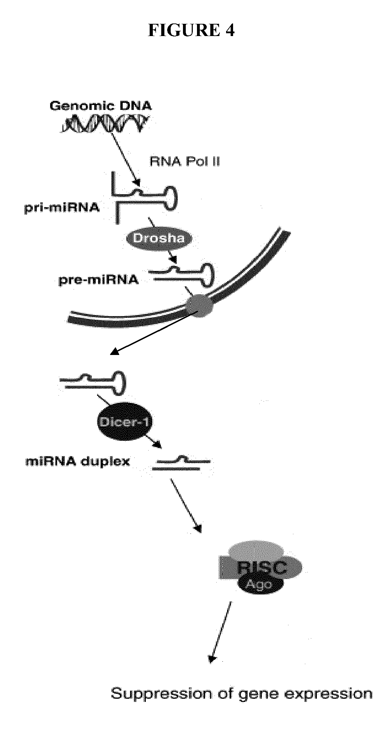

Figure 4 depicts transcription by Pol II through miRNA-encoding sequences, which results in the synthesis of primary miRNA transcripts (pri-miRNA) that form hairpin structures recognized by the miRNA-processing enzyme Drosha. The pri-miRNA is processed into pre-miRNA by Drosha and then exported to the cytoplasm, where they undergo further processing by Dicer to mature miRNA duplexes. These are un-wound by the RISC complexes and the mRNA-targeting strand is incorporated into RISC, which then targets mRNAs by either translational suppression or mRNA degradation, depending on miRNA-mRNA sequence complementarity. Adapted from van den Berg et al. Biochim. et Biophys. Acta 2008.

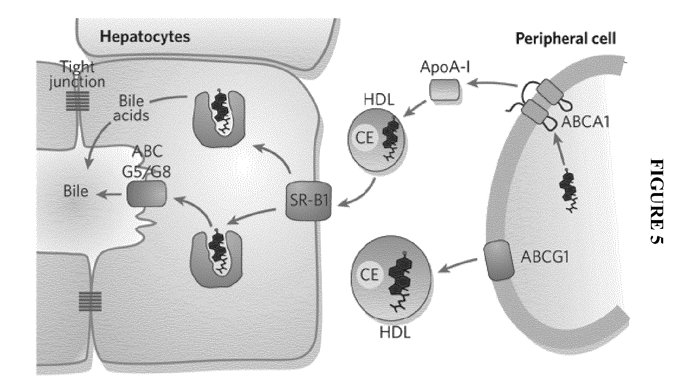

Figure 5 depicts the ABC transporter ABCA1 (and the related ABCG1), which exports free cholesterol out of peripheral cells, such as macrophages and atherogenic foam cells, to ApoA-I and High Density Lipoprotein (HDL). The HDLs bind to scavenger receptor-B1 (SR-B1) in hepatocytes, and transfer their associated cholesterol and cholesterol esters (CE) to the liver. The cholesterol is excreted into the bile either as free cholesterol or after conversion to bile salts. Adapted from Maxfield and Tabas. Nature 2005. 438:612-21.

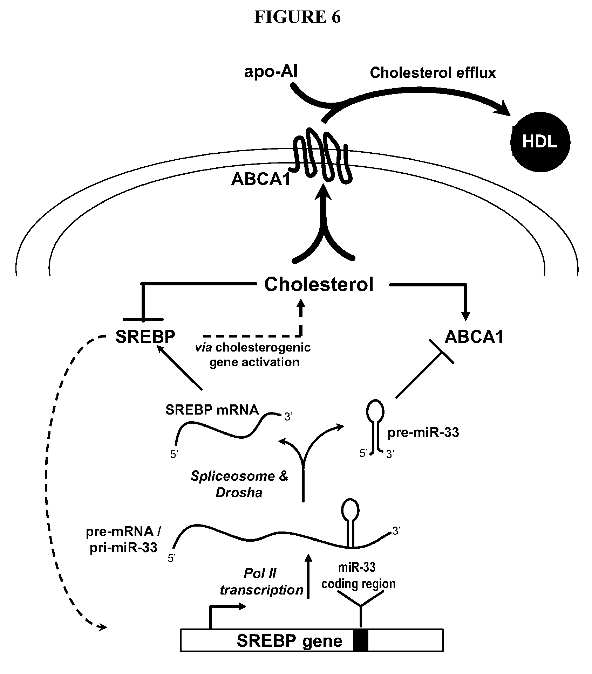

Figure 6 depicts a model for coordinated regulation of cholesterol homeostasis by miR-33 and its host gene product SREBP. miR-33 represses ABCA1 translation by targeting its 3' UTR. This step is proposed to be coordinated with the expression of SREBP, which activates cholesterol biosynthesis and uptake through transactivation of cholesterogenic genes. Cholesterol accumulation inhibits the SREBP pathway, and activates expression of ABCA1, which translocates to the cell membrane and mediates cholesterol efflux to ApoA-I and HDL.

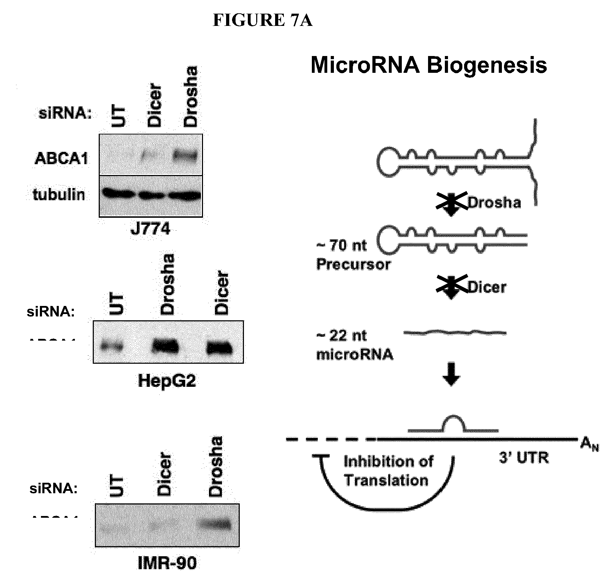

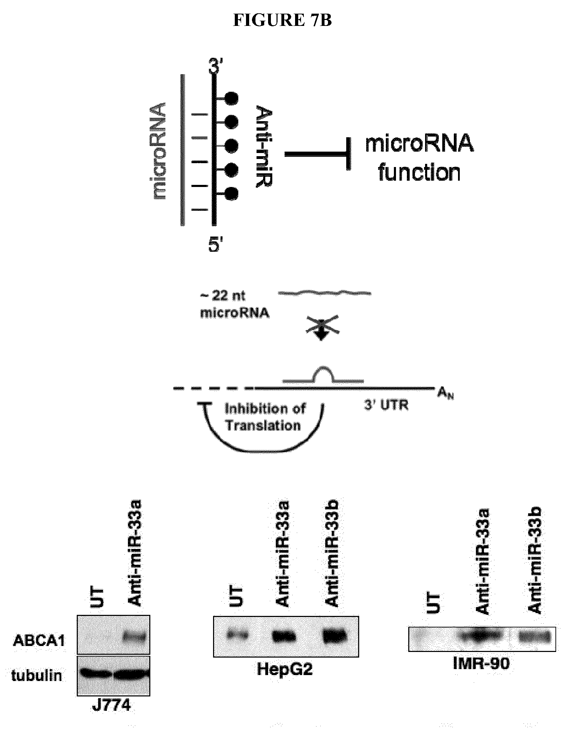

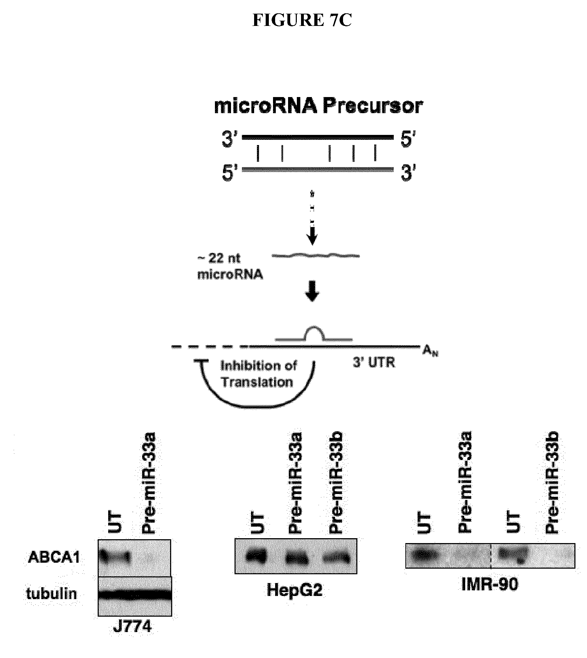

Figure 7 depicts regulation of ABCA1 expression by miRNAs. In Figure 7A, RNAi-directed knockdown of the miRNA processing enzymes Drosha and Dicer using siRNAs in three cell lines (J774 mouse macrophages, human HepG2 hepatocellular carcinoma cells, and normal human IMR-90 fibroblasts) resulted in an increase in ABCA1 protein levels. Protein levels were normalized by Bradford analysis. ß-Tubulin was used as loading control. In Figure 7B, antagonism of miR-33 function by anti-miR-33 antisense oligonucleotides is shown to result in higher expression of ABCA1. Figure 7C depicts the effect of miR-33, which is mimicked by transfecting cells with miRNA precursor Pre-miR-33. As expected, ABCA1 expression is inhibited through translational repression by increased levels of miR-33.

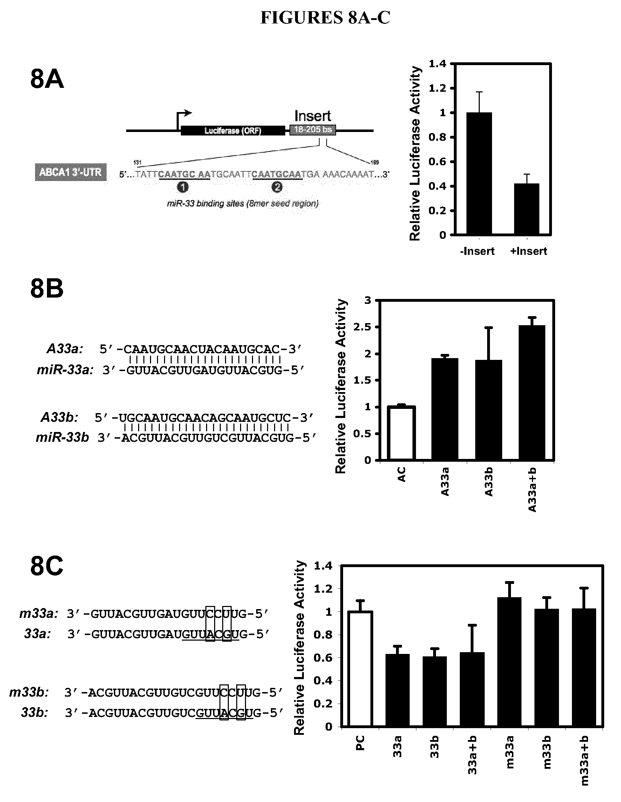

Figures 8A-C depict an evaluation of the predicted target sites for miR-33 at ABCA1 3'-UTR by Luciferase reporter constructs in HEK293 cells. (A) Two potential target sites are predicted for miR-33 which reside within the first 160 bases of the ABCA1 3' UTR (SEQ ID NO:5). Insertion of the corresponding sequence (bases 18-205) of the ABCA1 3' UTR downstream of the Luciferase ORF results in the down-regulation of Luciferase activity (right graph). (B) The down-regulation observed in (A) is blocked by sequence-specific depletion of the miR-33a/b isoforms (SEQ ID NOs:7 and 9) using Anti-microRNA oligonucleotides (Ambion) A33a and A33b (SEQ ID NOs:6 and 8) at a concentration of 30 nM (AC: unrelated Anti-miR as a negative control). (C) The addition of excess exogenous wild-type miR-33 Precursor (33a and 33b at 30 nM), but not mutated miR-33 Precursor (m33a (SEQ ID NO:10) and m33b (SEQ ID NO:11) at 30 nM), further inhibited the Luciferase activity as expected.

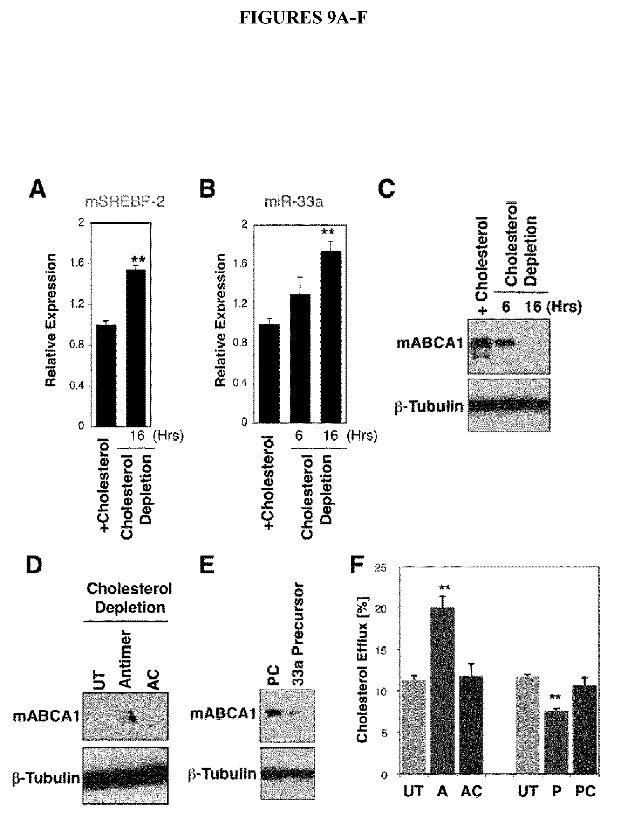

Figures 9A-F depicts reciprocal regulation by cholesterol and SREBP/miR-33. Lovastatin and β-cyclodextrin-mediated depletion of cholesterol in J774 mouse macrophages results in increased expression of (A) SREBP-2 and (B) miR-33a, and (C) in decreased ABCA1 protein levels. Error bars represent s.d. ** denotes p<0.01. (D) Cholesterol-depleted J774 mouse macrophages exhibit upregulation of ABCA1 protein in response to transfection with miR-33b antisense oligonucleotides as compared with mock transfection (UT) or antisense control (AC) oligonucleotides. β-tubulin served as loading control in this immunoblot. (E) Transfection of J774 macrophages cultured in the presence of serum/cholesterol with miR-33a precursor results in decreased levels of ABCA1 as compared with precursor control (PC). (F) Introduction of miR-33a antisense oligonucleotides (A) into J774 macrophages loaded with radio-labeled cholesterol results in increased cholesterol efflux, whereas miR-33a precursors (P) inhibit cholesterol efflux, as compared with transfection controls (UT) or scrambled antisense control (AC) or precursor control (PC) oligonucleotides. Error bars represent s.d. ** denotes p<0.01.

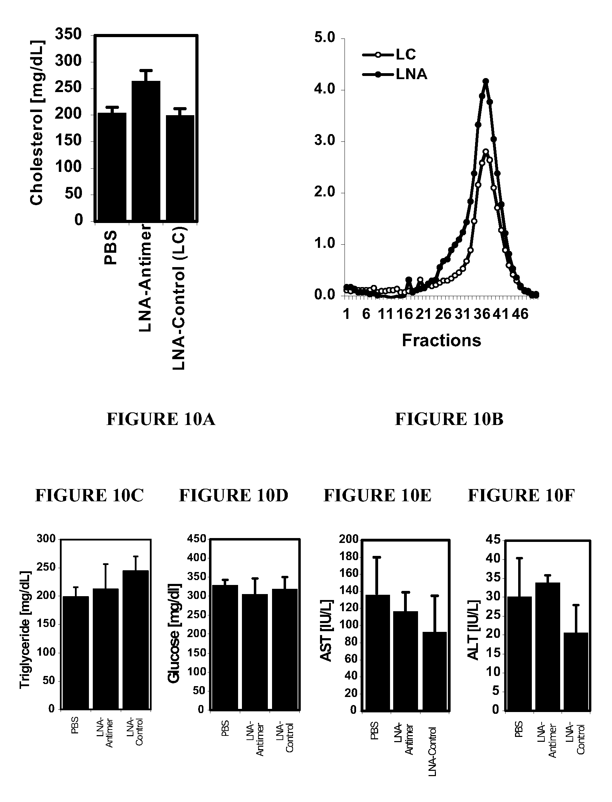

Figures 10A-G depict regulation of HDL by miR-33a in vivo. (A) Tail vein injection of mice fed a western-type diet with Locked Nucleic Acid (LNA)-antisense oligonucleotides directed against mouse miR-33a results in elevated total serum cholesterol in comparison with vehicle (PBS) and scramble LNA control (LC) oligonucleotides. (B) FPLC analysis of pooled serum from 5 mice on a western-type diet treated with LNA-miR-33a antisense (LNA) and LNA control (LC) revealed an increase in the magnitude of the HDL-cholesterol peak in the miR-33a antisense-treated animals. Elution of lipoprotein standards is indicated by the labels. Plasma triglycerides (C), glucose (D), AST (E), and ALT (F) were unaffected by the treatments. (G) Represents a model for coordinated regulation of cholesterol homeostasis by miR-33 and its host gene product SREBP.

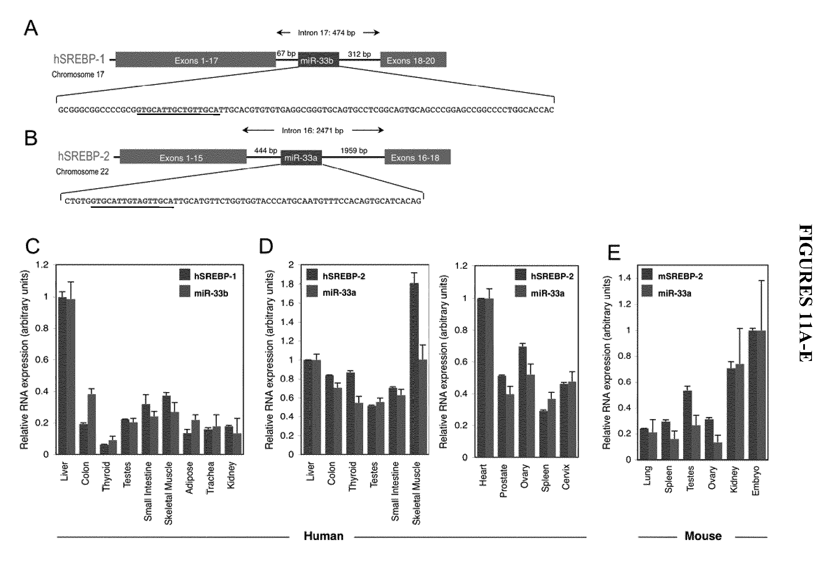

Figures 11A-E depict co-expression of SREBPs in human and mouse tissues. Human SREBP-1 (A; SEQ ID NO:12) and SREBP-2 (B; SEQ ID NO:13) genes harbor related intronic miRNAs (miR-33b and miR-33a, respectively). The sequences encoding the pri-miRNAs are shown, with the mature miRNA sequences underlined. (C-E) Correlation of expression of miR-33a/b and SREBP host genes in selected human and mouse tissues: (C) hSREBP-1/-hsa-miR-33b, (D) hSREBP-2/hsa-miR-33a, (E) mSREBP-1/mmu-miR-33b. Error bars represent s.e.m.

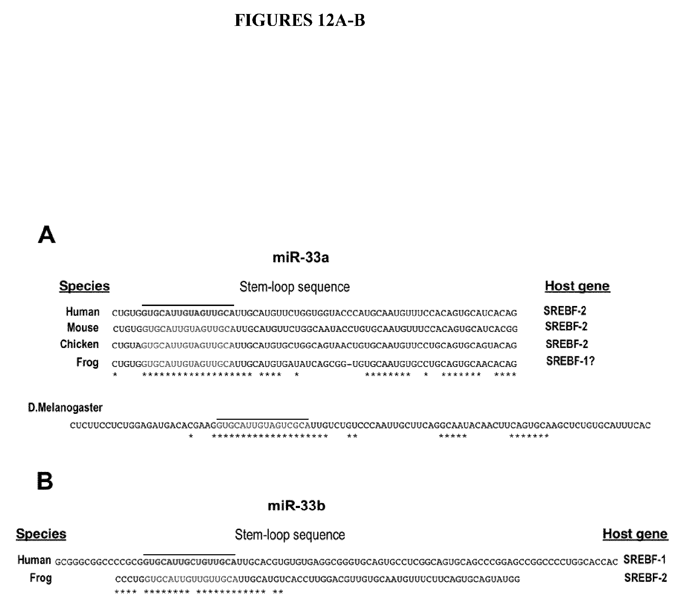

Figure 12 depicts the sequence and genomic context of miR-33a (A) and miR-33b (B) isoforms in selected species (mir-33a in human, SEQ ID NO:14; mouse, SEQ ID NO:15; chicken, SEQ ID NO:16; putative frog, SEQ ID NO:17, and Drosophila, SEQ ID NO:18; mir-33b in human, SEQ ID NO:19, in mouse, SEQ ID NO:20). The miRNA stem-loops are shown and grouped according to the SREBP genes in which they reside. Asterisks represent the positions of perfectly conserved residues.

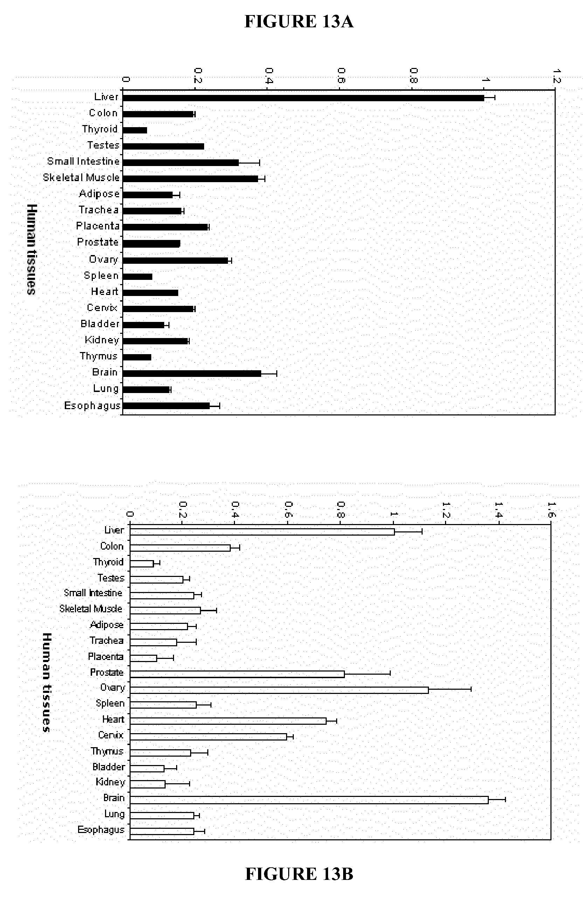

Figure 13A depicts relative expression of SREBP-1 in human tissues. β-Actin was used to normalize expression between tissues. (B) Relative expression of hsa-miR-33b in human tissues. U6 was used to normalize expression between tissues. Total RNA used from each tissue was pooled from at least three donors including male and female patients.

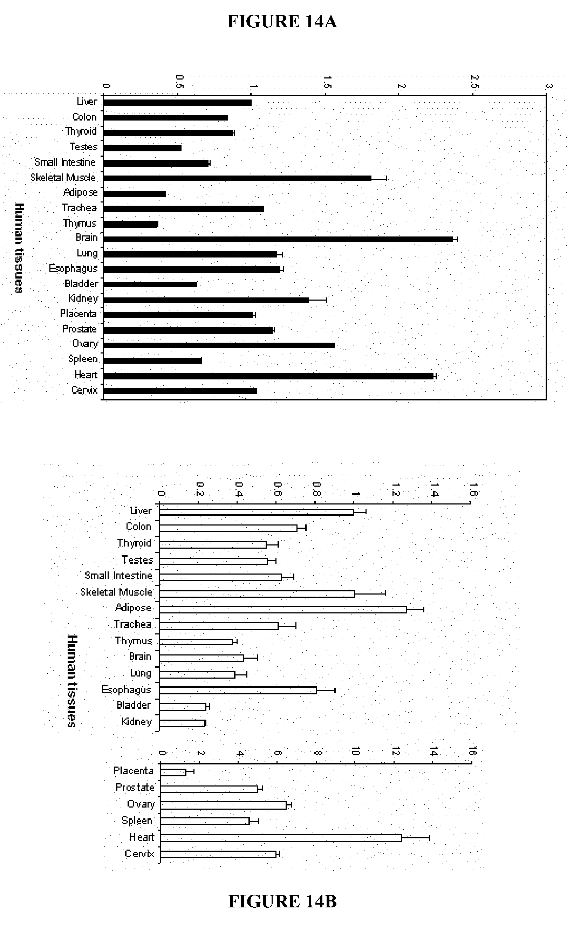

Figure 14A depicts relative expression of SREBP-2 in human tissues. β-Actin was used to normalize expression between tissues. (B) Relative expression of hsa-miR-33a in human tissues. U6 was used to normalize expression between tissues. Tissues with low relative expression are compared in the graph on the left, and tissues with high relative expression are compared in the graph on the right. Total RNA used from each tissue was pooled from at least three donors including male and female patients.

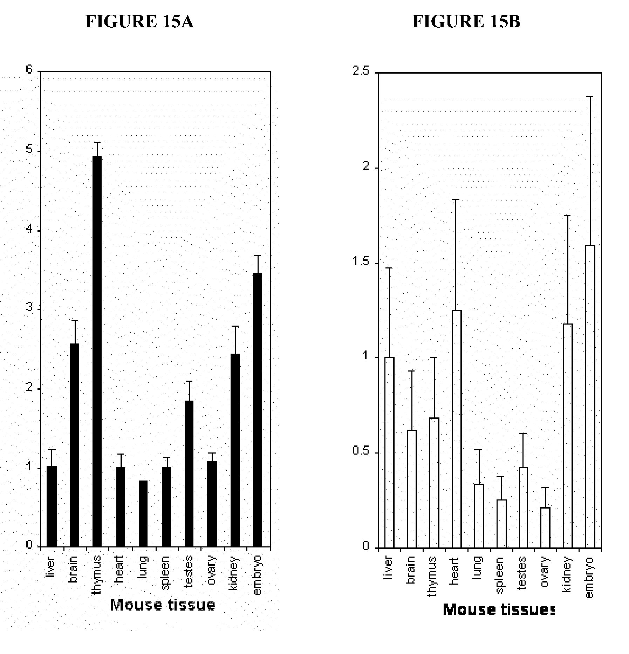

Figure 15A depicts the relative expression of SREBP-2 in mouse tissues. β-Actin was used to normalize expression between tissues. (B) Relative expression of mmu-miR-33a in mouse tissues. U6 was used to normalize expression between tissues.

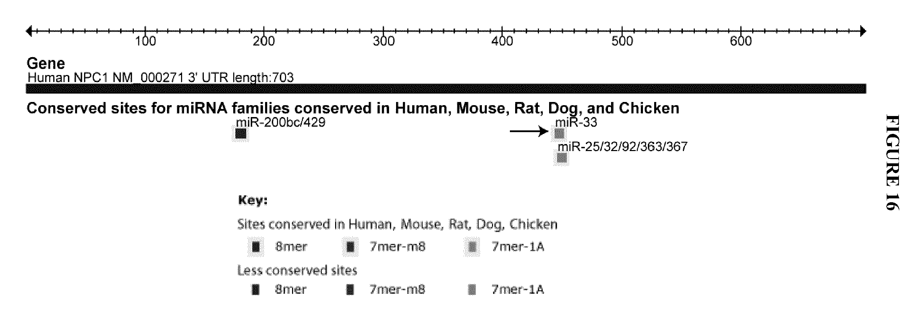

Figure 16 depicts the predicted miRNA target sites in the human NPC1 3' UTR (TargetScan 4.2). The miR-33 target site is indicated by an arrow.

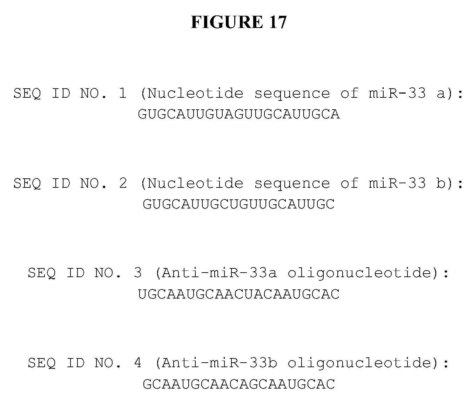

Figure 17 depicts miR-33 a and b, as SEQ ID NOs. 1 and 2, respectively. Also shown are Anti-miR-33a and b oligonucleotides, as SEQ ID NOs. 3 and 4, respectively.

### DETAILED DESCRIPTION OF THE INVENTION

### Definitions

Unless otherwise defined, all technical and scientific terms used herein have the same meaning as commonly understood by one of ordinary skill in the art to which this invention belongs. In case of conflict, the present application, including definitions, will control.

As referred to herein, a "complementary nucleic acid sequence" is a nucleic acid sequence capable of hybridizing with another nucleic acid sequence comprised of complementary nucleotide base pairs. By "hybridize" is meant pair to form a double-stranded molecule between complementary nucleotide bases (e.g., adenine (A) forms a base pair with thymine (T), as does guanine (G) with cytosine (C) in DNA) under suitable conditions of stringency. (See, e.g., Wahl, G. M. and S. L. Berger (1987) Methods Enzymol. 152:399; Kimmel, A. R. (1987) Methods Enzymol. 152:507).

As used herein, an "antisense oligonucleotide" refers to a synthesized nucleic acid sequence that is complementary to a DNA or mRNA sequence, such as that of a microRNA.

"RNA" is a molecule comprising at least one or more ribonucleotide residues. A "ribonucleotide" is a nucleotide with a hydroxyl group at the 2' position of a beta-D-ribofuranose moiety. The term RNA, as used herein, includes double-stranded RNA, single-stranded RNA, isolated RNA, such as partially purified RNA, essentially pure RNA, synthetic RNA, recombinantly produced RNA, as well as altered RNA that differs from naturally occurring RNA by the addition, deletion, substitution and/or alteration of one or more nucleotides. Nucleotides of the RNA molecules can also comprise non-standard nucleotides, such as non-naturally occurring nucleotides or chemically synthesized nucleotides or deoxynucleotides.

A "microRNA" (miRNA) typically refers to a single-stranded RNA molecules of about 21-23 nucleotides in length, which regulates gene expression. miRNAs are encoded by genes from whose DNA they are transcribed but miRNAs are not translated into protein; instead each primary transcript is processed into a short stem-loop structure before undergoing further processing into a functional miRNA. Mature miRNA molecules are partially complementary to one or more messenger RNA (mRNA) molecules, and their main function is to down-regulate gene expression.

As used herein "an interfering RNA" refers to any double stranded or single stranded RNA sequence, capable -- either directly or indirectly (i.e., upon conversion) -- of inhibiting or down regulating gene expression by mediating RNA interference. Interfering RNA includes but is not limited to small interfering RNA ("siRNA") and small hairpin RNA ("shRNA"). "RNA interference" refers to the selective degradation of a sequence-compatible messenger RNA transcript.

As used herein "an shRNA" (small hairpin RNA) refers to an RNA molecule comprising an antisense region, a loop portion and a sense region, wherein the sense region has complementary nucleotides that base pair with the antisense region to form a duplex stem. Following post-transcriptional processing, the small hairpin RNA is converted into a small interfering RNA by a cleavage event mediated by the enzyme Dicer, which is a member of the RNase III family.

A "small interfering RNA" or "siRNA" as used herein refers to any small RNA molecule capable of inhibiting or down regulating gene expression by mediating RNA interference in a sequence specific manner. The small RNA can be, for example, about 18 to 21 nucleotides long.

As used herein, an "antagomir" refers to a small synthetic RNA having complementarity to a specific microRNA target, with either mispairing at the cleavage site or one or more base modifications to inhibit cleavage.

As used herein, the phrase "post-transcriptional processing" refers to mRNA processing that occurs after transcription and is mediated, for example, by the enzymes Dicer and/or Drosha.

As used herein, the term "ABCA1" refers to the ATP-binding cassette, subfamily A, member 1, protein transporter, described, for example, by Remaley, A. T., Proc. Nat. Acad. Sci. 96: 12685-12690, 1999.

As used herein "an increase in ABCA1 protein expression" refers to an amount of ABCA1 protein that is at least about 1-fold more (for example 1, 2, 3, 4, 5, 10, 20, 30, 40, 50, 60, 70, 80, 90, 100, 1000, 10,000-fold or more) than the amount of ABCA1 protein in a subject prior to treatment according to the methods described herein. "Increased" as it refers to the amount of ABCA1 protein expression in a subject also means at least about 5% more (for example 5, 6, 7, 8, 9, 10, 15, 20, 25, 30, 35, 40, 45, 50, 55, 60, 65, 70, 75, 80, 85, 90, 95, 99 or 100%) than the amount of ABCA1 protein in the subject before treatment according to the methods described herein. Protein amounts can be measured according to methods known in the art.

By "an effective amount" is meant the amount of a required agent or composition comprising the agent to ameliorate the symptoms of a disease relative to an untreated patient. The effective amount of composition(s) used for therapeutic treatment of a disease varies depending upon the manner of administration, the age, body weight, and general health of the subject. Ultimately, the attending physician or veterinarian will decide the appropriate amount and dosage regimen. Such amount is referred to as an "effective" amount."

As used herein, "cholesterol homeostasis" refers to the regulation of cholesterol uptake, cholesterol biosynthesis, cholesterol conversion to bile acids and excretion of bile acids as such processes occur in a subject having healthful levels of LDL, HDL and cholesterol in the blood (e.g., such healthful levels are also referred to herein as a "reference standard"). Accordingly, a subject in need of cholesterol homeostasis is in need of improved regulation resulting in a return to healthful levels of LDL, HDL and/or cholesterol in the blood.

A "subject" is a vertebrate, including any member of the class mammalia, including humans, domestic and farm animals, and zoo, sports or pet animals, such as mouse, rabbit, pig, sheep, goat, cattle and higher primates.

As used herein, a "vector" or "expression vector" is a nucleic acid-based delivery vehicle comprising regulatory sequences and a gene of interest, which can be used to transfer its contents into a cell.

In this disclosure, "comprises," "comprising," "containing" and "having" and the like can have the meaning ascribed to them in U.S. Patent law and can mean " includes," "including," and the like; "consisting essentially of' or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

Other definitions appear in context throughout this disclosure.

### Compositions and Methods

MicroRNAs (miRNAs) are a class of small (e.g., 18-24 nucleotides) non-coding RNAs that exist in a variety of organisms, including mammals, and are conserved in evolution. miRNAs are processed from hairpin precursors of about 70 nucleotides which are derived from primary transcripts through sequential cleavage by the RNAse III enzymes drosha and dicer. Many microRNAs can be encoded in intergenic regions, hosted within introns of pre-mRNAs or within ncRNA genes. Many miRNAs also tend to be clustered and transcribed as polycistrons and often have similar spatial temporal expression patterns. MiRNAs have been found to have roles in a variety of biological processes including developmental timing, differentiation, apoptosis, cell proliferation, organ development, and metabolism.

MicroRNAs-33 a and b (miR-33 a and b), referred to herein as SEQ ID NOs. 1 and 2, respectively, target the ATP-binding cassette transporter ABCA1 for translational repression. Accordingly, inhibition of miR-33 represents a novel therapeutic means to increase ABCA1 levels, HDL production, and reverse cholesterol transport.

Nucleic acids for use according to the invention are designed to inhibit miR-33 a and/or b in a subject in need of cholesterol homeostasis. In one aspect, the invention provides a a nucleic acid sequence that is complementary to SEQ ID NOs. 1 or 2, for use according to the inventions in an amount effective to increase the expression of the ATP-binding cassette, subfamily A, member 1, (ABCA1) protein in a human subject in need of cholesterol homeostasis. This can be achieved, for example, by administering an antisense oligonucleotide that is complementary to miR-33 a or b, including but not limited to the antisense oligonucleotide of SEQ ID NO. 3 or SEQ ID NO. 4. Other nucleic acid sequences for use in according to the invention and that are complementary to miR-33 a or b can be those which inhibit post-transcriptional processing of miR-33 a or b, such as an interfering RNA, including but not limited to an shRNA or siRNA, or an antagomir.

Antisense oligonucleotides are typically designed to block expression of a DNA or RNA target by binding to the target and halting expression at the level of transcription, translation, or splicing. Antisense oligonucleotides for use according to the present invention are complementary nucleic acid sequences designed to hybridize under stringent conditions to miR-33 a and/or b. Thus, oligonucleotides are chosen that are sufficiently complementary to the target, i.e., that hybridize sufficiently well and with sufficient specificity, to give the desired effect.

In the context of this invention, hybridization means hydrogen bonding, which may be Watson-Crick, Hoogsteen or reversed Hoogsteen hydrogen bonding, between complementary nucleoside or nucleotide bases. For example, adenine and thymine are complementary nucleobases which pair through the formation of hydrogen bonds. Complementary, as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target.

It is understood in the art that a complementary nucleic acid sequence need not be 100% complementary to that of its target nucleic acid to be specifically hybridizable. A complementary nucleic acid sequence for use according to the invention is specifically hybridizable when binding of the sequence to the target DNA or RNA molecule interferes with the normal function of the target DNA or RNA to cause a loss of activity, and there is a sufficient degree of complementarity to avoid non-specific binding of the sequence to non-target sequences under conditions in which specific binding is desired, i.e., under physiological conditions in the case of in vivo assays or therapeutic treatment, and in the case of in vitro assays, under conditions in which the assays are performed under suitable conditions of stringency. For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30° C, more preferably of at least about 37° C, and most preferably of at least about 42° C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred embodiment, hybridization will occur at 30° C in 750 mM NaCl, 75 mM trisodium citrate, and 1% SDS. In a more preferred embodiment, hybridization will occur at 37° C in 500 mM NaCl, 50 mM trisodium citrate, 1% SDS, 35% formamide, and 100 µg/ml denatured salmon sperm DNA (ssDNA). In a most preferred embodiment, hybridization will occur at 42° C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25° C, more preferably of at least about 42° C, and even more preferably of at least about 68° C. In a preferred embodiment, wash steps will occur at 25° C in 30 mM NaCl, 3 mM trisodium citrate, and 0.1% SDS. In a more preferred embodiment, wash steps will occur at 42° C. in 15 mM NaCl, 1.5 mM trisodium citrate, and 0.1% SDS. In a more preferred embodiment, wash steps will occur at 68° C in 15 mM NaCl, 1.5 mM trisodium citrate, and 0.1% SDS. Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York.

It is preferred that the antisense oligonucleotides for use according to the present invention comprise at least 80% sequence complementarity to a target region within the target nucleic acid, moreover that they comprise 90% sequence complementarity and even more preferable to comprise 95% sequence complementarity to the target region within the target nucleic acid sequence to which they are targeted. For example, an antisense compound in which 18 of 20 nucleobases of the antisense oligonucleotide are complementary, and would therefore specifically hybridize, to a target region would represent 90 percent complementarity. Percent complementarity of an antisense compound with a region of a target nucleic acid can be determined routinely using basic local alignment search tools (BLAST programs) (Altschul et al., J. Mol. Biol., 1990, 215, 403-410; Zhang and Madden, Genome Res., 1997, 7, 649-656). Antisense and other compounds for use according to the invention, which hybridize to miR-33 a or b, are identified through experimentation, and representative sequences of these compounds are hereinbelow identified as preferred embodiments of the invention (e.g., including but not limited to the antisense oligonucleotide of SEQ ID NO. 3 or SEQ ID NO. 4).

In another embodiment, the nucleic acid sequence that is complementary to SEQ ID NOs. 1 or 2 can be an interfering RNA, including but not limited to an shRNA or siRNA. Interfering RNA includes, but is not limited to small interfering RNA ("siRNA") and small hairpin RNA ("shRNA"). Methods for constructing interfering RNAs are well known in the art. For example, the interfering RNA can be assembled from two separate oligonucleotides, where one strand is the sense strand and the other is the antisense strand, wherein the antisense and sense strands are self-complementary (i.e., each strand comprises nucleotide sequence that is complementary to nucleotide sequence in the other strand; such as where the antisense strand and sense strand form a duplex or double stranded structure); the antisense strand comprises nucleotide sequence that is complementary to a nucleotide sequence in a target nucleic acid molecule or a portion thereof (i.e., an undesired gene) and the sense strand comprises nucleotide sequence corresponding to the target nucleic acid sequence or a portion thereof. Alternatively, interfering RNA is assembled from a single oligonucleotide, where the self-complementary sense and antisense regions are linked by means of nucleic acid based or non-nucleic acid-based linker(s). The interfering RNA can be a polynucleotide with a duplex, asymmetric duplex, hairpin or asymmetric hairpin secondary structure, having self-complementary sense and antisense regions, wherein the antisense region comprises a nucleotide sequence that is complementary to nucleotide sequence in a separate target nucleic acid molecule or a portion thereof and the sense region having nucleotide sequence corresponding to the target nucleic acid sequence or a portion thereof. The interfering can be a circular single-stranded polynucleotide having two or more loop structures and a stem comprising self-complementary sense and antisense regions, wherein the antisense region comprises nucleotide sequence that is complementary to nucleotide sequence in a target nucleic acid molecule or a portion thereof and the sense region having nucleotide sequence corresponding to the target nucleic acid sequence or a portion thereof, and wherein the circular polynucleotide can be processed either in vivo or in vitro to generate an active siRNA molecule capable of mediating RNA interference.

In specific embodiments of the invention, the interfering RNA coding region encodes a self-complementary RNA molecule having a sense region, an antisense region and a loop region. Such an RNA molecule when expressed desirably forms a "hairpin" structure, and is referred to herein as an "shRNA." The loop region is generally between about 2 and about 10 nucleotides in length. In a preferred embodiment, the loop region is from about 6 to about 9 nucleotides in length. In one such embodiment of the invention, the sense region and the antisense region are between about 15 and about 20 nucleotides in length. Following post-transcriptional processing, the small hairpin RNA is converted into a siRNA by a cleavage event mediated by the enzyme Dicer, which is a member of the RNase III family. The siRNA is then capable of inhibiting the expression of a gene with which it shares homology. For details, see Brummelkamp et al., Science 296:550-553, (2002); Lee et al, Nature Biotechnol., 20, 500-505, (2002); Miyagishi and Taira, Nature Biotechnol 20:497-500, (2002); Paddison et al. Genes & Dev. 16:948-958, (2002); Paul, Nature Biotechnol, 20, 505-508, (2002); Sui, Proc. Natl. Acad. Sd. USA, 99(6), 5515-5520, (2002); Yu et al. Proc NatlAcadSci USA 99:6047-6052, (2002).

The target RNA cleavage reaction guided by siRNAs is highly sequence specific. In general, siRNA containing a nucleotide sequences identical to a portion of the target gene (i.e., miR-33 a and/or b) are preferred for inhibition. However, 100% sequence identity between the siRNA and the target gene is not required to practice the present invention. Thus the invention has the advantage of being able to tolerate sequence variations that might be expected due to genetic mutation, strain polymorphism, or evolutionary divergence. For example, siRNA sequences with insertions, deletions, and single point mutations relative to the target sequence have also been found to be effective for inhibition. Alternatively, siRNA sequences with nucleotide analog substitutions or insertions can be effective for inhibition.

In yet another embodiment, the nucleic acid sequence that is complementary to SEQ ID NOs. 1 or 2 is an antagomir. Antagomirs are single stranded, double stranded, partially double stranded and hairpin structured chemically modified oligonucleotides that target a microRNA. Preferably, an antagomir featured in the invention includes a nucleotide sequence sufficiently complementary to hybridize to a miRNA target sequence of about 12 to 25 nucleotides, preferably about 15 to 23 nucleotides.

In specific embodiments, antagomirs are RNA-like oligonucleotides that harbor various modifications for RNase protection and pharmacologic properties such as enhanced tissue and cellular uptake. An antagomir can differs from normal RNA by having complete 2'-O-methylation of sugar, phosphorothioate backbone and a cholesterol-moiety at 3'-end. Phosphorothioate modifications provide protection against RNase activity and their lipophilicity contributes to enhanced tissue uptake. In a preferred embodiment, the antagomir includes six phosphorothioate backbone modifications; two phosphorothioates are located at the 5'-end and four at the 3'-end. Antagomirs of the present invention can also be modified with respect to their length or otherwise the number of nucleotides making up the antagomir. It is preferred that the antagomirs of the present invention are about 20 - 21 nucleotides in length for optimal function, as this size matches the size of the mature microRNAs for miR-33 a and b.

The nucleic acid sequences for use according to the invention, whether RNA, cDNA, genomic DNA, vectors, viruses or hybrids thereof, can be isolated from a variety of sources, genetically engineered, amplified, and/or expressed/ generated recombinantly. Recombinant nucleic acid sequences can be individually isolated or cloned and tested for a desired activity. Any recombinant expression system can be used, including e.g. in vitro, bacterial, fungal, mammalian, yeast, insect or plant cell expression systems.

Nucleic acid sequences of the invention can be inserted into delivery vectors and expressed from transcription units within the vectors. The recombinant vectors can be DNA plasmids or viral vectors. Generation of the vector construct can be accomplished using any suitable genetic engineering techniques well known in the art, including, without limitation, the standard techniques of PCR, oligonucleotide synthesis, restriction endonuclease digestion, ligation, transformation, plasmid purification, and DNA sequencing, for example as described in Sambrook et al. Molecular Cloning: A Laboratory Manual. (1989)), Coffin et al. (Retroviruses. (1997)) and "RNA Viruses: A Practical Approach" (Alan J. Cann, Ed., Oxford University Press, (2000)). As will be apparent to one of ordinary skill in the art, a variety of suitable vectors are available for transferring nucleic acids of the invention into cells. The selection of an appropriate vector to deliver nucleic acids and optimization of the conditions for insertion of the selected expression vector into the cell, are within the scope of one of ordinary skill in the art without the need for undue experimentation. Viral vectors comprise a nucleotide sequence having sequences for the production of recombinant virus in a packaging cell. Viral vectors expressing nucleic acids of the invention can be constructed based on viral backbones including, but not limited to, a retrovirus, lentivirus, adenovirus, adeno-associated virus, pox virus or alphavirus. The recombinant vectors capable of expressing the nucleic acids of the invention can be delivered as described herein, and persist in target cells (e.g., stable transformants).

Nucleic acid sequences for use according to the invention can be synthesized in vitro by well-known chemical synthesis techniques, as described in, e.g., Adams (1983) J. Am. Chem. Soc. 105:661; Belousov (1997) Nucleic Acids Res. 25:3440-3444; Frenkel (1995) Free Radic. Biol. Med. 19:373-380; Blommers (1994) Biochemistry 33:7886-7896; Narang (1979) Meth. Enzymol. 68:90; Brown (1979) Meth. Enzymol. 68:109; Beaucage (1981) Tetra. Lett. 22:1859; U.S. Patent No. 4,458,066.

Nucleic acid sequences for use according to the invention can be stabilized against nucleolytic degradation such as by the incorporation of a modification, e.g., a nucleotide modification. For example, nucleic acid sequences of the invention includes a phosphorothioate at least the first, second, or third internucleotide linkage at the 5' or 3' end of the nucleotide sequence. As another example, the nucleic acid sequence can include a 2'-modified nucleotide, e.g., a 2'-deoxy, 2'-deoxy-2'-fluoro, 2'-O-methyl, 2'-O-methoxyethyl (2'-O-MOE), 2'-O-aminopropyl (2'-O-AP), 2'-O-dimethylaminoethyl (2'-O-DMAOE), 2'-O-dimethylaminopropyl (2'-O-DMAP), 2'-O-dimethylaminoethyloxyethyl (2'-O-DMAEOE), or 2'-O--N-methylacetamido (2'-O--NMA). As another example, the nucleic acid sequence can include at least one 2'-O-methyl-modified nucleotide, and in some embodiments, all of the nucleotides include a 2'-O-methyl modification. In some embodiments, the nucleic acids are "locked," i.e., comprise nucleic acid analogues in which the ribose ring is "locked" by a methylene bridge connecting the 2'-O atom and the 4'-C atom (see, e.g., Kaupinnen et al., Drug Disc. Today 2(3):287-290 (2005); Koshkin et al., J. Am. Chem. Soc., 120(50):13252-13253 (1998)).

Techniques for the manipulation of nucleic acids for use according to the invention, such as, e.g., subcloning, labeling probes (e.g., random-primer labeling using Klenow polymerase, nick translation, amplification), sequencing, hybridization and the like are well described in the scientific and patent literature, see, e.g., Sambrook, ed., MOLECULAR CLONING: A LABORATORY MANUAL (2ND ED.), Vols. 1-3, Cold Spring Harbor Laboratory, (1989); CURRENT PROTOCOLS IN MOLECULAR BIOLOGY, Ausubel, ed. John Wiley & Sons, Inc., New York (1997); LABORATORY TECHNIQUES IN BIOCHEMISTRY AND MOLECULAR BIOLOGY: HYBRIDIZATION WITH NUCLEIC ACID PROBES, Part I. Theory and Nucleic Acid Preparation, Tijssen, ed. Elsevier, N.Y. (1993).

Described herein are pharmaceutical compositions and formulations comprising nucleic acid sequences designed to target the ATP-binding cassette transporter ABCA1 for translational repression, thereby increasing ABCA1 levels, HDL production, and reverse cholesterol transport.

Pharmaceutical compositions can be formulated with a pharmaceutically acceptable carrier. Pharmaceutical compositions and formulations can be administered parenterally, topically, orally or by local administration, such as by aerosol or transdermally. Pharmaceutical compositions can be formulated in any way and can be administered in a variety of unit dosage forms depending upon the condition or disease (e.g., type of cardiovascular disorder) and the degree of illness, the general medical condition of each patient, the resulting preferred method of administration and the like. Details on techniques for formulation and administration of pharmaceuticals are well described in the scientific and patent literature, see, e.g., the latest edition of Remington's Pharmaceutical Sciences, Maack Publishing Co, Easton PA ("Remington's").

Nucleic acid sequences of the invention can be administered alone or as a component of a pharmaceutical formulation (composition). The compounds may be formulated for administration, in any convenient way for use in human or veterinary medicine. Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Pharmaceutical formulations include those suitable for intradermal, inhalation, oral/ nasal, topical, parenteral, rectal, and/or intravaginal administration. The formulations may conveniently be presented in unit dosage form and may be prepared by any methods well known in the art of pharmacy. The amount of active ingredient (e.g., nucleic acid sequences of this invention) which can be combined with a carrier material to produce a single dosage form will vary depending upon the host being treated, the particular mode of administration, e.g., intradermal or inhalation. The amount of active ingredient which can be combined with a carrier material to produce a single dosage form will generally be that amount of the compound which produces a therapeutic effect, e.g., an antigen specific T cell or humoral response.

Pharmaceutical formulations can be prepared according to any method known to the art for the manufacture of pharmaceuticals. Such drugs can contain sweetening agents, flavoring agents, coloring agents and preserving agents. A formulation can be admixtured with nontoxic pharmaceutically acceptable excipients which are suitable for manufacture. Formulations may comprise one or more diluents, emulsifiers, preservatives, buffers, excipients, etc. and may be provided in such forms as liquids, powders, emulsions, lyophilized powders, sprays, creams, lotions, controlled release formulations, tablets, pills, gels, on patches, in implants, etc.

Pharmaceutical formulations for oral administration can be formulated using pharmaceutically acceptable carriers well known in the art in appropriate and suitable dosages. Such carriers enable the pharmaceuticals to be formulated in unit dosage forms as tablets, pills, powder, dragees, capsules, liquids, lozenges, gels, syrups, slurries, suspensions, etc., suitable for ingestion by the patient. Pharmaceutical preparations for oral use can be formulated as a solid excipient, optionally grinding a resulting mixture, and processing the mixture of granules, after adding suitable additional compounds, if desired, to obtain tablets or dragee cores. Suitable solid excipients are carbohydrate or protein fillers include, e.g., sugars, including lactose, sucrose, mannitol, or sorbitol; starch from corn, wheat, rice, potato, or other plants; cellulose such as methyl cellulose, hydroxypropylmethyl-cellulose, or sodium carboxy-methylcellulose; and gums including arabic and tragacanth; and proteins, e.g., gelatin and collagen. Disintegrating or solubilizing agents may be added, such as the crosslinked polyvinyl pyrrolidone, agar, alginic acid, or a salt thereof, such as sodium alginate. Push-fit capsules can contain active agents mixed with a filler or binders such as lactose or starches, lubricants such as talc or magnesium stearate, and, optionally, stabilizers. In soft capsules, the active agents can be dissolved or suspended in suitable liquids, such as fatty oils, liquid paraffin, or liquid polyethylene glycol with or without stabilizers.

Aqueous suspensions can contain an active agent (e.g., nucleic acid sequences of the invention) in admixture with excipients suitable for the manufacture of aqueous suspensions, e.g., for aqueous intradermal injections. Such excipients include a suspending agent, such as sodium carboxymethylcellulose, methylcellulose, hydroxypropylmethylcellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension can also contain one or more preservatives such as ethyl or n-propyl p-hydroxybenzoate, one or more coloring agents, one or more flavoring agents and one or more sweetening agents, such as sucrose, aspartame or saccharin. Formulations can be adjusted for osmolarity.

Oil-based pharmaceuticals can be used for administration of nucleic acid sequences of the invention. Oil-based suspensions can be formulated by suspending an active agent in a vegetable oil, such as arachis oil, olive oil, sesame oil or coconut oil, or in a mineral oil such as liquid paraffin; or a mixture of these. See e.g., U.S. Patent No. 5,716,928 describing using essential oils or essential oil components for increasing bioavailability and reducing inter- and intra-individual variability of orally administered hydrophobic pharmaceutical compounds (see also U.S. Patent No. 5,858,401). The oil suspensions can contain a thickening agent, such as beeswax, hard paraffin or cetyl alcohol. Sweetening agents can be added to provide a palatable oral preparation, such as glycerol, sorbitol or sucrose. These formulations can be preserved by the addition of an antioxidant such as ascorbic acid. As an example of an injectable oil vehicle, see Minto (1997) J. Pharmacol. Exp. Ther. 281:93-102.

Pharmaceutical formulations can also be in the form of oil-in-water emulsions. The oily phase can be a vegetable oil or a mineral oil, described above, or a mixture of these. Suitable emulsifying agents include naturally-occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion can also contain sweetening agents and flavoring agents, as in the formulation of syrups and elixirs. Such formulations can also contain a demulcent, a preservative, or a coloring agent. Alternatively, these injectable oil-in-water emulsions can comprise a paraffin oil, a sorbitan monooleate, an ethoxylated sorbitan monooleate and/or an ethoxylated sorbitan trioleate.

Pharmaceutical compounds can be administered by in intranasal, intraocular and intravaginal routes including suppositories, insufflation, powders and aerosol formulations (for examples of steroid inhalants, see e.g., Rohatagi (1995) J. Clin. Pharmacol. 35:1187-1193; Tjwa (1995) Ann. Allergy Asthma Immunol. 75:107-111). Suppositories formulations can be prepared by mixing the drug with a suitable non-irritating excipient which is solid at ordinary temperatures but liquid at body temperatures and will therefore melt in the body to release the drug. Such materials are cocoa butter and polyethylene glycols.

Pharmaceutical compounds can be delivered transdermally, by a topical route, formulated as applicator sticks, solutions, suspensions, emulsions, gels, creams, ointments, pastes, jellies, paints, powders, and aerosols.

Pharmaceutical compounds can also be delivered as microspheres for slow release in the body. For example, microspheres can be administered via intradermal injection of drug which slowly release subcutaneously; see Rao (1995) J. Biomater Sci. Polym. Ed. 7:623-645; as biodegradable and injectable gel formulations, see, e.g., Gao (1995) Pharm. Res. 12:857-863 (1995); or, as microspheres for oral administration, see, e.g., Eyles (1997) J. Pharm. Pharmacol. 49:669-674.

Pharmaceutical compounds can be parenterally administered, such as by intravenous (IV) administration or administration into a body cavity or lumen of an organ. These formulations can comprise a solution of active agent dissolved in a pharmaceutically acceptable carrier. Acceptable vehicles and solvents that can be employed are water and Ringer's solution, an isotonic sodium chloride. In addition, sterile fixed oils can be employed as a solvent or suspending medium. For this purpose any bland fixed oil can be employed including synthetic mono- or diglycerides. In addition, fatty acids such as oleic acid can likewise be used in the preparation of injectables. These solutions are sterile and generally free of undesirable matter. These formulations may be sterilized by conventional, well known sterilization techniques. The formulations may contain pharmaceutically acceptable auxiliary substances as required to approximate physiological conditions such as pH adjusting and buffering agents, toxicity adjusting agents, e.g., sodium acetate, sodium chloride, potassium chloride, calcium chloride, sodium lactate and the like. The concentration of active agent in these formulations can vary widely, and will be selected primarily based on fluid volumes, viscosities, body weight, and the like, in accordance with the particular mode of administration selected and the patient's needs. For IV administration, the formulation can be a sterile injectable preparation, such as a sterile injectable aqueous or oleaginous suspension. This suspension can be formulated using those suitable dispersing or wetting agents and suspending agents. The sterile injectable preparation can also be a suspension in a nontoxic parenterally-acceptable diluent or solvent, such as a solution of 1,3-butanediol. The administration can be by bolus or continuous infusion (e.g., substantially uninterrupted introduction into a blood vessel for a specified period of time).

Pharmaceutical compounds and formulations can be lyophilized. A stable lyophilized formulation comprising a composition can be made by lyophilizing a solution comprising a pharmaceutical and a bulking agent, e.g., mannitol, trehalose, raffinose, and sucrose or mixtures thereof. A process for preparing a stable lyophilized formulation can include lyophilizing a solution about 2.5 mg/mL protein, about 15 mg/mL sucrose, about 19 mg/mL NaCl, and a sodium citrate buffer having a pH greater than 5.5 but less than 6.5. See, e.g., U.S. patent app.no.20040028670.

Compositions and formulations can be delivered by the use of liposomes. By using liposomes, particularly where the liposome surface carries ligands specific for target cells, or are otherwise preferentially directed to a specific organ, one can focus the delivery of the active agent into target cells *in vivo.* See, e.g., U.S. Patent Nos. 6,063,400; 6,007,839; Al-Muhammed (1996) J. Microencapsul. 13:293-306; Chonn (1995) Curr. Opin. Biotechnol. 6:698-708; Ostro (1989) Am. J. Hosp. Pharm. 46:1576-1587.

Formulations can be administered for prophylactic and/or therapeutic treatments. For therapeutic applications, compositions are administered to a subject in need of cholesterol homeostasis in an amount sufficient to cure, alleviate or partially arrest the clinical manifestations of the disorder or its complications, e.g., cardiovascular defects; this can be called a therapeutically effective amount. For example, pharmaceutical compositions can be administered in an amount sufficient to increase the expression of the ATP-binding cassette, subfamily A, member 1, (ABCA1) protein, increase efflux of intracellular cholesterol and/or production of HDL in the liver and/or decrease the amount of cholesterol circulating the blood of a subject in need of cholesterol homeostasis.

The amount of pharmaceutical composition adequate to accomplish this is a therapeutically effective dose. The dosage schedule and amounts effective for this use, i.e., the dosing regimen, will depend upon a variety of factors, including the stage of the disease or condition, the severity of the disease or condition, the general state of the patient's health, the patient's physical status, age and the like. In calculating the dosage regimen for a patient, the mode of administration also is taken into consideration.

The dosage regimen also takes into consideration pharmacokinetics parameters well known in the art, i.e., the active agents' rate of absorption, bioavailability, metabolism, clearance, and the like (see, e.g., Hidalgo-Aragones (1996) J. Steroid Biochem. Mol. Biol. 58:611-617; Groning (1996) Pharmazie 51:337-341; Fotherby (1996) Contraception 54:59-69; Johnson (1995) J. Pharm. Sci. 84:1144-1146; Rohatagi (1995) Pharmazie 50:610-613; Brophy (1983) Eur. J. Clin. Pharmacol. 24:103-108; the latest Remington's, supra). The state of the art allows the clinician to determine the dosage regimen for each individual patient, active agent and disease or condition treated. Guidelines provided for similar compositions used as pharmaceuticals can be used as guidance to determine the dosage regiment, i.e., dose schedule and dosage levels, administered practicing the methods of the invention are correct and appropriate.

Single or multiple administrations of formulations can be given depending on for example: the dosage and frequency as required and tolerated by the patient, the degree and amount of cholesterol homeostasis generated after each administration, and the like. The formulations should provide a sufficient quantity of active agent to effectively treat, prevent or ameliorate conditions, diseases or symptoms, e.g., increase the expression of ABCA1, increase efflux of intracellular cholesterol and/or production of HDL in the liver and/or decrease the amount of cholesterol circulating the blood of a subject in need of cholesterol homeostasis.

Pharmaceutical formulations for oral administration can be in a daily amount of between about 1 to 100 or more mg per kilogram of body weight per day. Lower dosages can be used, in contrast to administration orally, into the blood stream, into a body cavity or into a lumen of an organ. Substantially higher dosages can be used in topical or oral administration or administering by powders, spray or inhalation. Actual methods for preparing parenterally or non-parenterally administrable formulations will be known or apparent to those skilled in the art and are described in more detail in such publications as Remington's, supra.

Various studies have reported successful mammalian dosing using complementary nucleic acid sequences. For example, Esau C., et al., (2006) Cell Metabolism, 3(2):87-98 reported dosing of normal mice with intraperitoneal doses of miR-122 antisense oligonucleotide ranging from 12.5 to 75 mg/kg twice weekly for 4 weeks. The mice appeared healthy and normal at the end of treatment, with no loss of body weight or reduced food intake. Plasma transaminase levels were in the normal range (AST ¾ 45, ALT ¾ 35) for all doses with the exception of the 75 mg/kg dose of miR-122 ASO, which showed a very mild increase in ALT and AST levels. They concluded that 50mg/kg was an effective, nontoxic dose. Another study by Krützfeldt J., et al., (2005) Nature 438, 685-689, injected anatgomirs to silence miR-122 in mice using a total dose of 80, 160 or 240 mg per kg body weight. The highest dose resulted in a complete loss of miR-122 signal. In yet another study, locked nucleic acids ("LNAs") were successfully applied in primates to silence miR-122. Elmen J., et al., (2008) Nature 452, 896-899, report that efficient silencing of miR-122 was achieved in primates by three doses of 10 mg kg-1 LNA-antimiR, leading to a long-lasting and reversible decrease in total plasma cholesterol without any evidence for LNA-associated toxicities or histopathological changes in the study animals.

Methods described herein further comprise co-administration with other drugs or pharmaceuticals, e.g., compositions for providing cholesterol homeostasis. For example, compositions and formulations described herein are co-administered with drugs for treating elevated LDL cholesterol such as HMG CoA reductase inhibitors, e.g., atorvastatin, fluvastatin, lovastatin, pravastatin, rosuvastatin and simvastatin; bile acid sequestrants, e.g., colesevelam, cholestyramine and colestipol; and nicotinic acid (niacin).

The present invention is additionally described by way of the following illustrative, non-limiting Examples that provide a better understanding of the present invention and of its many advantages.

### EXAMPLES

Herein, a series of in vitro and in vivo studies to further delineate the role of miR-33 in controlling cholesterol homeostasis in cooperation with SREBPs are described. The following examples are put forth for illustrative purposes only and are not intended to limit the scope of what the inventors regard as their invention.

### Example 1: Predicted targets of miR-33 in humans and mice

Among available approaches to the identification of mRNA targets by miRNA, computational prediction studies have been shown to represent useful methods in establishing the first steps towards experimental validation of miRNA targets. The most critical parameter considered in predicting miRNA targets is the ability of the miRNA sequence to undergo specific base pairing with its cognate target, known as "seed pairing" (Bartel DP (2004) Cell 116: 281-297; van den Berg A, Mols J, Han J (2008) Biochim Biophys Acta.). This involves 7-8 bases of the miRNA 5'-end. In this context, among a list of more than 100 predicted targets derived from both Miranda and TargetScan 4.2 target prediction databases (microrna.sanger.ac.uk; www.targetscan.org/vert_42/), the most relevant conserved target for miR-33 in both humans and mice is ABCA1 (Table 1 and Fig. 7). Interestingly, these studies revealed that additional proteins that are known to play a role in the cholesterol trafficking network or lipid homeostasis and which are functionally associated with ABCA1 also may serve as targets both in humans and mice. These include Niemann-Pick disease, type Cl (NPC1) (Table 1).

**Table 1. Predicted human miR-33 targets by Miranda and TargetScan 4.2 software programs. ABCA1 and NPC1 are highlighted in bold/underlined.**

| *Human 33a-Miranda* | *Human 33b-Miranda* | *Human 33b-TargetScan* |
|---|---|---|
| HADHB | HADHB | **ABCA1** |
| CROT | CROT | B3GALT2 |
| **ABCA1** | **ABCA1** | CDK6 |
| PXMP2 | PXMP2 | HMGA2 |
| TAF12 | TAF 12 | GLCCI1 |
| PGM1 | PGM1 | NUFIP2 |
| LOC727902 | LOC727902 | ATP8B1 |
| HMX3 | RDH8 | SLC17A6 |
| PCM1 | HMX3 | SCN8A |
| DNAJC12 | PCM1 | MRPS25 |
| DGKH | DGKH | CUL5 |
| HAGH | HAGH | ZRANB2 |
| ZNF281 | ZNF281 | NSF |
| C20orf103 | C20orf103 | AFF2 |
| MYOCD | MYOCD | ABHD2 |
| CHN2 | SLC10A7 | VCPIP1 |
| CSMD1 | CHN2 | MMP16 |
| ICT1 | ICT1 | ANKRD57 |
| HIPK2 | DEPDC6 | SLC25A25 |
| CHAC2 | HIPK2 | KCNA4 |
| SAT1 | CHAC2 | SATB2 |
| C11orf58 | SAT1 | DYNC1LI2 |
| **NPC1** | **NPC1** | RAP2A |
| C16orf80 | SCN10VA | SLC12A5 |
| FGFBP1 | C16orf80 | KCNMA1 |
| BTBD2 | FGFBP1 | SETD7 |
| LAMA3 | BMP3 | EN2 |
| C6orf213 | BTBD2 | ERBB2IP |
| FGF3 | LAMA3 | ZNF281 |
| MPP1 | FGF3 | GRIA3 |
| IRX2 | MPP1 | **NPC1** |

### Example 2: Down-regulation of ABCA1 by miR-33 in human and mouse cell lines

To validate the results from the bioinformatics prediction studies, a set of experiments were carried out using human and mouse cell lines, including human HepG2 hepatocellular carcinoma cells, IMR-90 primary human fibroblasts, and the mouse macrophage cell line J774, as model systems. These cell lines exhibit low expression of ABCA1 protein at low cell densities. Translation inhibition of ABCA1 by miR-33 was expected to contribute to this low level expression. Therefore, siRNA knockdown of components of the miRNA biogenesis pathway was conducted to determine whether ABCA1 protein expression is regulated by miRNAs. Indeed, transfection with siRNAs directed against the Drosha and Dicer miRNA processing enzymes resulted in a substantial increase in ABCA1 protein expression (especially with Drosha siRNA) in all three cell lines examined (Fig. 7A). These results are consistent with regulation of ABCA1 by miRNAs.

To demonstrate that this upregulation results specifically from a lack of miR-33 processing, the three different cell lines were transfected with Anti-miR-33a/b oligonucleotides (Ambion). Anti-miRs are modified miRNA antisense oligonucleotides that block endogenous miRNA function in a sequence-specific manner by base-pairing. Both Anti-miR-33a and b transfections resulted in considerably higher expression of ABCA1 protein in human cell lines, as well as upon Anti-miR-33a treatment of J774 mouse macrophages (Fig. 7B).

These data indicate regulation of ABCA1 mRNA translation or stability by endogenous miR-33. To further evaluate the effect of miR-33 on ABCA1 expression, cells were transfected with synthetic Pre-miR-33s (Ambion) to increase the intra-cellular levels of miR-33a and b, respectively. As ABCA1 protein levels are difficult to detect 24 hrs after cell plating, the effects of Pre-miR-33s were evaluated 48 hrs after transfection and post-plating. The experiments clearly show that ABCA1 expression can be specifically repressed by miR-33, especially in mouse J774 macrophages and the human IMR-90 fibroblasts (Fig. 7C).

Recent studies have shown that ABCA1 works together with another cholesterol transporter, ABCG1, in macrophages to promote effective cholesterol efflux and HDL biogenesis (Fig. 5) (Tall AR, et al. (2008) Cell Metab 7: 365-375; Wang X, et al. (2007) J Clin Invest 117: 2216-2224). Indeed, data has indicated that when one transporter is deficient, the other is induced, suggesting compensatory regulation (Yvan-Charvet L, et al. (2007) J Clin Invest 117: 3900-3908; Ranalletta M, et al. (2006) Arterioscler Thromb Vasc Biol 26: 2308-2315). It was therefore investigated whether ABCG1 expression is altered upon changes in ABCA1 levels after manipulation of the miRNA pathway and miR-33a. Interestingly, no significant effect on ABCG1 expression by RNAi of Dicer and Drosha, anti-miR-33a transfection, or Pre-miR-33a expression in the mouse macrophage cell line J774 was observed. These findings suggest that at least under these preliminary experimental conditions, the expression of ABCA1 and ABCG1 are not reciprocally linked in response to miR-33 manipulations.

### Example 3: Targeting of the ABCA1 3' UTR by miR-33

To determine whether miR-33 affect ABCA1 mRNA and/or protein levels, Pre-miR-33a/b will be expressed in several cell lines (i.e. IMR-90, HepG2, and J774) to increase miR-33 levels, and Anti-miR-33 oligonucleotides will be transfected to antagonize miR-33, followed by analysis of ABCA1 mRNA and protein levels by quantitative RT-PCR and immunoblotting, respectively. Actin mRNA (qRT-PCR) and Tubulin protein (immunoblotting) will be used as controls.

In order to establish whether miR-33 affect ABCA1 levels through targeting of the 3' UTR, fragments of the human and mouse ABCA1 3' UTR harboring the predicted miR-33 target sequences were isolated and cloned into the 3' UTR cloning site of a Luciferase reporter plasmid (pMIR-REPORT, Ambion). The resulting ABCA1 3' UTR Luciferase reporters were transfected into human HEK293 cells, in the presence of miR-33a/b anti-miRs, precursors, or control oligonucleotides. As shown in Fig. 8A, insertion of a fragment of the ABCA1 3' UTR harboring the predicted miR-33 target sites into the Luciferase reporter vector resulted in a significant decrease in reporter activity as compared with vector without insert, consistent with the presence of inhibitory sequences within the inserted fragment (e.g. miR-33 recognition sites). Importantly, co-transfection with anti-miR oligonucleotides complementary to human miR-33a/b caused increased expression of the reporter, suggesting that endogenous miR-33a/b indeed target the ABCA1 3' UTR fragment in human HEK293 cells (Fig. 8B). Conversely, co-transfection with wild-type human miR-33a/b Precursor oligonucleotides (Ambion) caused further repression of reporter activity, while Precursor oligonucleotides mutated in the seed basepairing region had no effect, as compared with Precursor control oligonucleotides (Fig. 8C). The relative Luciferase activity was measured from absolute Luciferase activities normalized by Renilla Luciferase activity. (PC: unrelated microRNA Precursor as a negative control).

Together, these data offer important support for the view that miR-33a/b inhibit expression of human ABCA1 by targeting the ABCA1 3' UTR for translation repression or mRNA degradation.

### Example 4: Role for miR-33 in cholesterol trafficking from macrophages in mice

SREBPs are regulated in a classic negative feedback manner by cholesterol (Brown MS, Goldstein JL (1997) Cell 89: 331-340). The biomedical and physiological relevance of the miR-33/SREBP/ABCA1 cholesterol regulatory circuit was investigated using the mouse J774 macrophage model (de la Llera-Moya M, et al. (2010, Epub ahead of print) Arterioscler. Thromb. Vasc. Biol.). J774 mouse macrophages were subjected to cholesterol treatment for SREBP-expression modulation as follows: 150K cells were plated per well in a 24-well plate format and treated with 10 µg/ml cholesterol and 1 ug/ml of 25-Hydroxycholesterol (Sigma) for 48 hours. To activate SREBP expression, cells were then washed twice with PBS and the media replaced by DMEM containing 10% lipid-depleted FBS, 5 mM (2-Hydroxypropyl)-β-cyclodextrin (Sigma) and 50 uM Lovastatin (Mevinolin, Sigma) and harvested at indicated time points. Both cholesterol-treated and -depleted cells were harvested at the same time points. Depletion of cholesterol by lovastatin/β-cyclodextrin treatment resulted in increased expression of both miR-33a and the mSREBP-2 host gene in J774 macrophages, with a concomitant decrease in ABCA1 protein expression (Fig. 9A-C). The strong suppression of ABCA1 protein levels in response to cholesterol depletion was at least partially reversed by miR-33a antisense oligonucleotides (Fig. 9D), consistent with the notion that miR-33 mediates cholesterol-regulated post-transcriptional control of ABCA1 levels in mouse macrophages. High protein levels of ABCA1 observed in J774 macrophages cultured in the presence of serum/cholesterol were substantially suppressed by additional miR-33a precursor (Fig. 9E), in keeping with miR-33 regulation of ABCA1. This miRNA-mediated mechanism of ABCA1 regulation by cholesterol is in addition to the previously described transcriptional control of ABCA1 expression by oxysterols via the liver X receptor (LXR) (Tontonoz P, Mangelsdorf D, (2003) Mol. Endocrinol. 17:985), indicating multiple levels of cellular regulation of ABCA1 by cholesterol. Accordingly, mouse miR-33a and the SREBP-2 host gene are co-regulated by cholesterol, and suggest the existence of a reciprocal regulatory network of SREBP, miR-33, ABCA1, and cholesterol in macrophages.

Next, the effects of miR-33 manipulations on cholesterol efflux from J774 macrophages were examined. Cells were first labeled with [3H]-cholesterol and then transfected with miR-33a precursor, miR-33a antisense, or control oligonucleotides, followed by measurements of the level of [3H]-cholesterol efflux to serum/apoA-1. Briefly, low passage J774 cells were transfected by Anti-miR or Pre-microRNA duplex and plated in 24-well plates at 300K cells/well and [3H]-cholesterol was added. After 20 hours of incubation, cells were washed twice with PBS at room temperature and the media replaced by fresh media containing 15 µg/ml free apoA-I. After 2 or 4 hours, aliquots of the medium were removed, and the [3H]-cholesterol was measured by liquid scintillation counting. The [3H]-cholesterol present in the cells was determined by extracting the cells in sodium hydroxide and measuredfollowed by by liquid scintillation counting. As shown in Fig. 9F, introduction of miR-33a antisense oligonucleotides (A) into mouse macrophages caused a marked (twofold) increase in [3H]-cholesterol efflux, whereas miR-33a precursor (P) treatment led to a significant decrease in [3H]-cholesterol efflux, as compared with control oligonucleotides (AC/PC) or mock transfected cells (UT). These findings demonstrate that miR-33 post-transcriptional repression of ABCA1 results in retention of intracellular cholesterol in macrophages, and are consistent with miR-33 regulation of cholesterol trafficking through control of ABCA1 levels.

### Example 5: Manipulation of ABCA1 levels by miR-33 antisense in a mammalian in vivo model

Manipulation of ABCA1 levels by miR-33 antisense approaches also leads to augmented HDL-cholesterol levels in a mammalian in vivo model. Tail vein injections were performed on mice fed a western-type diet (i.e., supplemented with 42% kcal from milkfat) for 6 weeks prior to and during treatment using PBS (vehicle), LNA-miR-33a antisense, or LNA-control oligonucleotides. Blood was obtained through submandibular bleeding from the experimental mice for the cholesterol/triglyceride profile before the start of the treatment. To conduct the injections, 20 mg/kg/day miR-33-LNA or Control-LNA was dissolved in PBS (a total volume of 200 µl) and administered for three consecutive days through daily tail vein injections at the same time each day. Mice were sacrificed 48 hours after the last tail vein injection. Following injections over 5 days, mice were sacrificed and serum was collected as follows. Upon sacrifice, a total of 1 mL of blood was obtained from mice by right ventricular puncture. Blood was centrifuged at 8,000 rpm for 5 minutes to obtain serum and was frozen at -80°C. Total serum cholesterol, triglycerides, glucose, alanine aminotranferase (ALT) and aspartate aminotransferase (AST) were determined before and after the treatment with miR-33-LNA or Control-LNA through Heska Dri-Chem 4000 Chemistry Analyzer (Heska, Loveland, CO) at Massachusetts General Hospital, Center for Comparative Medicine, Diagnostic Laboratory. FPLC analysis of pooled serum was carried out as described (Hyogo H, et al. (2002) J. Biol. Chem. 277: 34117). Plasma HDL-cholesterol concentrations were significantly increased in LNA-miR-33a antisense-treated animals as compared to LNA-control-treated mice (Fig. 10A, B and Table 2). By contrast, there were no significant effects on plasma concentrations of LDL-cholesterol, triglycerides, or glucose levels (Fig. 10B-D and Table 2). In these experiments, there was also no apparent hepatotoxicity (plasma AST/ALT) (Fig. 10E, F and Table 2). The observed elevation in plasma HDL-cholesterol in response to LNA-miR-33a antisense treatment is consistent with regulation of ABCAI-dependent cholesterol efflux by miR-33a in vivo.

These results further confirm that the mammalian SREBP family of transcription factors, key regulators of cholesterogenic and lipogenic genes, are hosts to conserved miRNAs (miR-33a/b) that function in concert with the SREBP host gene products to govern intracellular cholesterol levels and cholesterol homeostasis in vertebrates. Fig. 10G depicts a model for coordinated regulation of cholesterol homeostasis by miR-33 and its host gene product SREBP. miR-33 represses ABCA1 translation by targeting its 3' UTR. This step is coordinated with the expression of SREBP, which activates cholesterol biosynthesis and uptake through transactivation of cholesterogenic genes. Cholesterol accumulation inhibits the SREBP pathway, and activates expression of ABCA1, which translocates to the cell membrane and mediates cholesterol efflux to apoA-I and HDL.

**Table 2**

| n | treatment | Cholesterol [mg/dL] | | Triglyceride [mg/dL] | | Glucose [mg/dL] | | ALT [IU/L] | | AST [IU/L] | |
|---|---|---|---|---|---|---|---|---|---|---|---|
| | | *before* | *after* | *before* | *after* | *before* | *after* | *before* | *after* | *before* | *after* |
| 1 | PBS | 163.5 | 167 | 211 | 211 | 159 | 318 | 17 | 68 | 58 | 101 |
| 2 | PBS | 239.5 | 209 | 372 | 195 | 193 | 333 | 16 | 33 | 39 | 27 |
| 3 | PBS | 190.5 | 220 | 141 | 256 | 226 | 329 | 37 | 10 | 35 | 135 |
| 4 | PBS | 230 | 222 | 257 | 171 | 186 | 378 | 14 | 25 | 44 | 289 |
| 5 | PBS | 189.5 | 205 | 263 | 161 | 201 | 282 | 52 | 14 | 39 | 135 |
| 1 | LNA | 243 | 325 | - | 249 | 236 | 214 | 26 | 17 | 60 | 67 |
| 2 | LNA | 188 | 204 | 329 | 193 | 216 | 421 | 46 | 61 | 37 | 209 |
| 3 | LNA | 234.5 | 264 | 278 | 283 | 264 | 264 | 44 | 23 | 68 | 44 |
| 4 | LNA | 239 | 247 | 486 | 204 | 218 | 391 | 35 | 35 | 63 | 33 |
| 5 | LNA | 215.5 | 280 | 289 | 133 | 158 | 230 | 28 | 33 | 153 | 229 |
| 1 | LC | 206 | 157 | - | 308 | 224 | 275 | 72 | 26 | 128 | 142 |
| 2 | LC | 214.5 | 224 | 243 | 241 | 390 | 365 | 21 | 22 | 30 | 71 |
| 3 | LC | 180 | 223 | 384 | 134 | 160 | 233 | 18 | 22 | 54 | 150 |
| 4 | LC | 201 | 206 | 197 | 167 | 233 | 416 | 26 | 15 | 67 | 46 |
| 5 | LC | 210 | 186 | 238 | 373 | 286 | 299 | 47 | 17 | 68 | 52 |

Table 2. Treatment of mice on a western-type diet with Locked Nucleic Acid (LNA)-miR-33a antimer. Total serum cholesterol (A), Plasma triglycerides (B), glucose (C), AST (D), and ALT (E) were measured in mice fed a western-type diet prior to and after injection with PBS (vehicle), LNA-antisense oligonucleotides directed against mouse miR-33a, and scramble LNA control (LC) oligonucleotides.

As depicted in these Examples, members of the mammalian SREBP family of transcription factors, key regulators of cholesterogenic and lipogenic genes, are hosts to conserved miRNAs (miR-33a/b) that function in concert with the SREBP host gene products to govern intracellular cholesterol levels and cholesterol homeostasis in vertebrates. miR-33 exerts post-transcriptional control of the ABCA1 cholesterol transporter, with important consequences for cholesterol trafficking in vitro and HDL synthesis in vivo.

### Prophetic Example 6: Targeting of NPC1 by miR-33

Niemann-Pick type CI protein (NPC 1) plays an important role in mobilization of cholesterol/lipids from late endosomes/lysosomes, via the trans-Golgi network (TGN) to the plasma membrane. This process is thought to be coordinated with mobilization of phospholipids and cholesterol to apoA-I and other apolipoproteins at the cell surface mediated by ABCA1 to generate nascent HDL. The 3' UTR of NPC1 harbors one predicted miR-33 target site (7-mer; TargetScan 4.2). Because of its known role in intracellular cholesterol trafficking and recent functional linkage to ABCA1 and cholesterol efflux, it is a candidate target for miR-33.

First, it will be determined whether miRNAs control NPC1 levels in the three cell lines described above by use of siRNA targeting the miRNA processing enzymes Drosha and Dicer. Second, Pre-miR-33 and Anti-miR-33 oligonucleotides will be introduced into these cell lines to address the specific role of miR-33 in NPC1 regulation. NPC1 protein levels will be determined by immunoblotting, with ß-tubulin as control. Any effects of miR-33 manipulations on the NPC1 RNA levels will be assessed by qRT-PCR. If effects on NCP1 protein and/or mRNA levels are observed after miR-33 manipulations, the NPC1 3' UTR harboring the predicted miR-33 targeting sequence will be cloned into the pMIR-REPORT vector, followed by miR-33 target site mutation, and Luciferase expression analysis as described herein above for ABCA1. To establish the potential role of NPC1 in cholesterol trafficking/efflux, the effects of siRNA targeting of NPC1 in the J774 mouse macrophage cell line on cholesterol efflux will be examined using the methods described above for ABCA1.

### REFERENCES

1. Maxfield FR, Tabas I (2005) Role of cholesterol and lipid organization in disease. Nature 438: 612-621.
2. Moller DE, Kaufman KD (2005) Metabolic syndrome: a clinical and molecular perspective. Annu Rev Med 56: 45-62.
3. Bengoechea-Alonso MT, Ericsson J (2007) SREBP in signal transduction: cholesterol metabolism and beyond. Curr Opin Cell Biol 19: 215-222.
4. Brown MS, Goldstein JL (1997) The SREBP pathway: regulation of cholesterol metabolism by proteolysis of a membrane-bound transcription factor. Cell 89: 331-340.
5. Eberle D, et al. (2004) SREBP transcription factors: master regulators of lipid homeostasis. Biochimie 86: 839-848.
6. Horton JD, Goldstein JL, Brown MS (2002) SREBPs: activators of the complete program of cholesterol and fatty acid synthesis in the liver. J Clin Invest 109: 1125-1131.
7. Raghow R, et al. (2008) SREBPs: the crossroads of physiological and pathological lipid homeostasis. Trends Endocrinol Metab 19: 65-73.
8. Ericsson J, Edwards PA (1998) CBP is required for sterol-regulated and sterol regulatory element-binding protein-regulated transcription. J Biol Chem 273: 17865-17870.
9. Näär AM, et al. (1998) Chromatin, TAFs, and a novel multiprotein coactivator are required for synergistic activation by Sp1 and SREBP-1a in vitro. Genes Dev. 12: 3020-3031.
10. Näär AM, et al. (1999) Composite coactivator ARC mediates chromatin-directed transcriptional activation. Nature 398: 828-832.
11. Näär AM, Lemon BD, Tjian R (2001) Transcriptional coactivator complexes. Annu Rev Biochem 70: 475-501.
12. Näär AM, et al. (2002) Human CRSP interacts with RNA polymerase II CTD and adopts a specific CTD-bound conformation. Genes Dev 16: 1339-1344.
13. Oliner JD AJ, Hansen SK, Zhou S, Tjian R. (1996) SREBP transcriptional activity is mediated through an interaction with the CREB-binding protein. Genes Dev 10: 2903-2911.
14. Taatjes DJ, et al. (2002) Structure, function, and activator-induced conformations of the CRSP coactivator. Science 295: 1058-1062.
15. Yang F, et al. (2006) An ARC/Mediator subunit required for SREBP control of cholesterol and lipid homeostasis. Nature 442: 700-704.
16. Bartel DP (2004) MicroRNAs: genomics, biogenesis, mechanism, and function. Cell 116: 281-297.
17. He L, Hannon GJ (2004) MicroRNAs: small RNAs with a big role in gene regulation. Nat Rev Genet 5: 522-531.
18. Krutzfeldt J, Stoffel M (2006) MicroRNAs: a new class of regulatory genes affecting metabolism. Cell Metab 4: 9-12.
19. Lewis BP, et al. (2003) Prediction of mammalian microRNA targets. Cell 115: 787-798.
20. Lee YS, Dutta A (2008) MicroRNAs in Cancer. Annu Rev Pathol.
21. Mattes J, Collison A, Foster PS (2008) Emerging role of microRNAs in disease pathogenesis and strategies for therapeutic modulation. Curr Opin Mol Ther 10: 150-157.
22. Thum T, Catalucci D, Bauersachs J (2008) MicroRNAs: novel regulators in cardiac development and disease. Cardiovasc Res 79: 562-570.
23. Stefani G, Slack FJ (2008) Small non-coding RNAs in animal development. Nat Rev Mol Cell Biol 9: 219-230.
24. Sonkoly E, Stahle M, Pivarcsi A (2008) MicroRNAs and immunity: novel players in the regulation of normal immune function and inflammation. Semin Cancer Biol 18: 131-140.
25. van den Berg A, Mols J, Han J (2008) RISC-target interaction: Cleavage and translational suppression. Biochim Biophys Acta.
26. Vasudevan S, Tong Y, Steitz JA (2007) Switching from repression to activation: microRNAs can up-regulate translation. Science 318: 1931-1934.
27. Lagos-Quintana M, Rauhut R, Lendeckel W, Tuschl T (2001) Identification of novel genes coding for small expressed RNAs. Science 294: 853-858.
28. Rodriguez A, Griffiths-Jones S, Ashurst JL, Bradley A (2004) Identification of mammalian microRNA host genes and transcription units. Genome Res 14: 1902-1910.
29. Elmen J, et al. (2008) LNA-mediated microRNA silencing in non-human primates. Nature 452: 896-899.
30. Elmen J, et al. (2008) Antagonism of microRNA-122 in mice by systemically administered LNA-antimiR leads to up-regulation of a large set of predicted target mRNAs in the liver. Nucleic Acids Res 36: 1153-1162.
31. Esau C, et al. (2006) miR-122 regulation of lipid metabolism revealed by in vivo antisense targeting. Cell Metab 3: 87-98.
32. Krutzfeldt J, et al. (2005) Silencing of microRNAs in vivo with 'antagomirs'. Nature 438: 685-689.
33. Tall AR, et al. (2008) HDL, ABC transporters, and cholesterol efflux: implications for the treatment of atherosclerosis. Cell Metab 7: 365-375.
34. Wang X, Rader DJ (2007) Molecular regulation of macrophage reverse cholesterol transport. Curr Opin Cardiol 22: 368-372.
35. Singaraja RR, et al. (2002) Increased ABCA1 activity protects against atherosclerosis. J Clin Invest 110: 35-42.
36. Wang X, et al. (2007) Macrophage ABCA1 and ABCG1, but not SR-BI, promote macrophage reverse cholesterol transport in vivo. J Clin Invest 117: 2216-2224.
37. Van Eck M, et al. (2006) Macrophage ATP-binding cassette transporter A1 overexpression inhibits atherosclerotic lesion progression in low-density lipoprotein receptor knockout mice. Arterioscler Thromb Vasc Biol 26: 929-934.
38. van Eck M, et al. (2002) Leukocyte ABCA1 controls susceptibility to atherosclerosis and macrophage recruitment into tissues. Proc Natl Acad Sci U S A 99: 6298-6303.
39. Koldamova R, Lefterov I (2007) Role of LXR and ABCA1 in the pathogenesis of Alzheimer's disease - implications for a new therapeutic approach. Curr Alzheimer Res 4: 171-178.
40. Wahrle SE, et al. (2008) Overexpression of ABCA1 reduces amyloid deposition in the PDAPP mouse model of Alzheimer disease. J Clin Invest 118: 671-682.
41. Brunham LR, Kruit JK, Verchere CB, Hayden MR (2008) Cholesterol in islet dysfunction and type 2 diabetes. J Clin Invest 118: 403-408.
42. Chawla A, et al. (2001) A PPAR gamma-LXR-ABCA1 pathway in macrophages is involved in cholesterol efflux and atherogenesis. Mol Cell 7: 161-171.
43. Venkateswaran A, et al. (2000) Control of cellular cholesterol efflux by the nuclear oxysterol receptor LXR alpha. Proc Natl Acad Sci U S A 97: 12097-12102.
44. Chisholm JW, Hong J, Mills SA, Lawn RM (2003) The LXR ligand T0901317 induces severe lipogenesis in the db/db diabetic mouse. J Lipid Res 44: 2039-2048.
45. Millatt LJ, Bocher V, Fruchart JC, Staels B (2003) Liver X receptors and the control of cholesterol homeostasis: potential therapeutic targets for the treatment of atherosclerosis. Biochim Biophys Acta 1631: 107-118.
46. Schmitz G, Langmann T (2005) Transcriptional regulatory networks in lipid metabolism control ABCA1 expression. Biochim Biophys Acta 1735: 1-19.
47. Tamehiro N, et al. (2007) Sterol regulatory element-binding protein-2- and liver X receptor-driven dual promoter regulation of hepatic ABC transporter A1 gene expression: mechanism underlying the unique response to cellular cholesterol status. J Biol Chem 282: 21090-21099.
48. Wong J, Quinn CM, Brown AJ (2006) SREBP-2 positively regulates transcription of the cholesterol efflux gene, ABCA1, by generating oxysterol ligands for LXR. Biochem J 400: 485-491.
49. Zeng L, et al. (2004) Sterol-responsive element-binding protein (SREBP) 2 down-regulates ATP-binding cassette transporter A1 in vascular endothelial cells: a novel role of SREBP in regulating cholesterol metabolism. J Biol Chem 279: 48801-48807.
50. Haidar B, et al. (2002) cAMP induces ABCA1 phosphorylation activity and promotes cholesterol efflux from fibroblasts. J Lipid Res 43: 2087-2094.
51. See RH, et al. (2002) Protein kinase A site-specific phosphorylation regulates ATP-binding cassette A1 (ABCA1)-mediated phospholipid efflux. J Biol Chem 277: 41835-41842.
52. Lu R, et al. (2008) ApoA-I facilitates ABCA1 recycle/accumulation to cell surface by inhibiting its intracellular degradation and increases HDL generation. Arterioscler Thromb Vasc Biol 28: 1820-1824.
53. Taylor AJ (2008) Evidence to support aggressive management of high-density lipoprotein cholesterol: implications of recent imaging trials. Am J Cardiol 101: 36B-43B.
54. Yvan-Charvet L, et al. (2007) Combined deficiency of ABCA1 and ABCG1 promotes foam cell accumulation and accelerates atherosclerosis in mice. J Clin Invest 117: 3900-3908.
55. Ranalletta M, et al. (2006) Decreased atherosclerosis in low-density lipoprotein receptor knockout mice transplanted with Abcg1-/- bone marrow. Arterioscler Thromb Vasc Biol 26: 2308-2315.
56. deGoma EM, deGoma RL, Rader DJ (2008) Beyond high-density lipoprotein cholesterol levels evaluating high-density lipoprotein function as influenced by novel therapeutic approaches. J Am Coll Cardiol 51: 2199-2211.
57. Neufeld EB, et al. (2004) The ABCA1 transporter modulates late endocytic trafficking: insights from the correction of the genetic defect in Tangier disease. J Biol Chem 279: 15571-15578.
58. Wang MD, et al. (2007) Differential regulation of ATP binding cassette protein A1 expression and ApoA-1 lipidation by Niemann-Pick type C1 in murine hepatocytes and macrophages. J Biol Chem 282: 22525-22533.
59. Boadu E, Francis GA (2006) The role of vesicular transport in ABCA1-dependent lipid efflux and its connection with NPC pathways. J Mol Med 84: 266-275.
60. Baek D, et al. (2008) The impact of microRNAs on protein output. Nature 455: 64-71.
61. Baskerville S, Bartel DP (2005) Microarray profiling of microRNAs reveals frequent coexpression with neighboring miRNAs and host genes. Rna 11: 241-247.
62. Foufelle F, Ferre P (2002) New perspectives in the regulation of hepatic glycolytic and lipogenic genes by insulin and glucose: a role for the transcription factor sterol regulatory element binding protein-1c. Biochem J 366: 377-391.
63. Foretz M, et al. (1999) ADD1/SREBP-1c is required in the activation of hepatic lipogenic gene expression by glucose. Mol Cell Biol 19: 3760-3768.
64. Radhakrishnan A, et al. (2007) Sterol-regulated transport of SREBPs from endoplasmic reticulum to Golgi: oxysterols block transport by binding to Insig. Proc Natl Acad Sci U S A 104: 6511-6518.
65. Repa JJ, et al. (2000) Regulation of mouse sterol regulatory element-binding protein-1c gene (SREBP-1c) by oxysterol receptors, LXRalpha and LXRbeta. Genes Dev 14: 2819-2830.
66. Hannah VC, et al. (2001) Unsaturated fatty acids down-regulate srebp isoforms 1a and 1c by two mechanisms in HEK-293 cells. J Biol Chem 276: 4365-4372.
67. Ou J, et al. (2001) Unsaturated fatty acids inhibit transcription of the sterol regulatory element-binding protein-1c (SREBP-1c) gene by antagonizing ligand-dependent activation of the LXR. Proc Natl Acad Sci U S A 98: 6027-6032.
68. Sheng Z, Otani H, Brown MS, Goldstein JL (1995) Independent regulation of sterol regulatory element-binding proteins 1 and 2 in hamster liver. Proc Natl Acad Sci U S A 92: 935-938.
69. Akinc A, et al. (2008) A combinatorial library of lipid-like materials for delivery of RNAi therapeutics. Nat Biotechnol 26: 561-569.
70. de la Llera-Moya M, et al. (2010, Epub ahead of print) Arterioscler. Thromb. Vasc. Biol.
   72. Tontonoz P, Mangelsdorf D, (2003) Mol. Endocrinol. 17:985.
71. Hyogo H, Roy S, Paigen B, Cohen D, (2002) J. Biol. Chem. 277: 34117.

## Claims

1. A nucleic acid that is complementary to SEQ ID NOs. 1 or 2, for use in the therapeutic treatment of a subject in need of cholesterol homeostasis by decreasing the amount of cholesterol circulating in the blood of the subject; or by increasing efflux of intracellular cholesterol and/or production of HDL in the liver of the subject.

2. A nucleic acid that is complementary to SEQ ID NOs. 1 or 2 for use according to claim 1, wherein the subject in need of cholesterol homeostasis has a cardiovascular defect.

3. A nucleic acid that is complementary to SEQ ID NOs. 1 or 2 for use according to claim 1 or 2, wherein the use increases the expression of the ATP-binding cassette, subfamily A, member 1, (ABCA1) protein in the subject.

4. A nucleic acid that is complementary to SEQ ID NOs. 1 or 2 for use according to claim 3, wherein the nucleic acid is SEQ ID NO. 3.

5. A nucleic acid that is complementary to SEQ ID NOs. 1 or 2 for use according to claim 3, wherein the nucleic acid is SEQ ID NO. 4.

6. A nucleic acid that is complementary to SEQ ID NOs. 1 or 2 for use according to any one of claims 1 - 5, which is an antagomir.

7. A nucleic acid that is complementary to SEQ ID NOs. 1 or 2 for use according to any one of claims 1 - 5, which is an antisense oligonucleotide.

8. A nucleic acid that is complementary to SEQ ID NOs. 1 or 2 for use according to any one of claims 1 - 7, which includes at least one 2'-O-methyl-modified nucleotide.

9. A nucleic acid that is complementary to SEQ ID NOs. 1 or 2 for use according to any one of claims 1 - 3, which is a small interfering RNA (siRNA) or small hairpin RNA.

10. A nucleic acid that is complementary to SEQ ID NOs. 1 or 2 for use according to any one of claims 1 - 9, wherein the nucleic acid can inhibit post-transcriptional processing of SEQ ID NO. 1.

11. A nucleic acid that is complementary to SEQ ID NOs. 1 or 2 for use according to any one of claims 1 - 9, wherein the nucleic acid can inhibit post-transcriptional processing of SEQ ID NO. 2.

## Patentansprüche

1. Nucleinsäure, die zu Seq.-ID Nr. 1 oder 2 komplementär ist, zur Verwendung zur therapeutischen Behandlung eines Individuums mit Bedarf an Cholesterinhomöostase durch Reduktion der Menge des im Blut des Individuums zirkulierenden Cholesterins; oder durch Erhöhung des Ausflusses von intrazellulärem Cholesterin und/oder der Produktion von HDL in der Leber des Individuums.

2. Nucleinsäure, die zu Seq.-ID Nr. 1 oder 2 komplementär ist, zur Verwendung nach Anspruch 1, worin das Individuum mit Bedarf an Cholesterinhomöostase einen Herz-Gefäß-Defekt aufweist.

3. Nucleinsäure, die zu Seq.-ID Nr. 1 oder 2 komplementär ist, zur Verwendung nach Anspruch 1 oder 2, worin die Verwendung die Expression des Proteins der ATP-Bindungskassette, Unterfamilie A, Mitglied 1 (ABCA1-Protein), in dem Individuum erhöht.

4. Nucleinsäure, die zu Seq.-ID Nr. 1 oder 2 komplementär ist, zur Verwendung nach Anspruch 3, worin die Nucleinsäure Seq.-ID Nr. 3 ist.

5. Nucleinsäure, die zu Seq.-ID Nr. 1 oder 2 komplementär ist, zur Verwendung nach Anspruch 3, worin die Nucleinsäure Seq.-ID Nr. 4 ist.

6. Nucleinsäure, die zu Seq.-ID Nr. 1 oder 2 komplementär ist, zur Verwendung nach einem der Ansprüche 1 bis 5, die ein Antagomir ist.

7. Nucleinsäure, die zu Seq.-ID Nr. 1 oder 2 komplementär ist, zur Verwendung nach einem der Ansprüche 1 bis 5, die ein Antisense-Oligonucleotid ist.

8. Nucleinsäure, die zu Seq.-ID Nr. 1 oder 2 komplementär ist, zur Verwendung nach einem der Ansprüche 1 bis 7, die zumindest ein 2'-O-Methyl-modifiziertes Nucleotid umfasst.

9. Nucleinsäure, die zu Seq.-ID Nr. 1 oder 2 komplementär ist, zur Verwendung nach einem der Ansprüche 1 bis 3, die Small-interfering-RNA (siRNA) oder Smallhairpin-RNA ist.

10. Nucleinsäure, die zu Seq.-ID Nr. 1 oder 2 komplementär ist, zur Verwendung nach einem der Ansprüche 1 bis 9, worin die Nucleinsäure die posttranskriptionale Prozessierung von Seq.-ID Nr. 1 hemmen kann.

11. Nucleinsäure, die zu Seq.-ID Nr. 1 oder 2 komplementär ist, zur Verwendung nach einem der Ansprüche 1 bis 9, worin die Nucleinsäure die posttranskriptionale Prozessierung von Seq.-ID Nr. 2 hemmen kann.

## Revendications

1. Acide nucléique qui est complémentaire aux SEQ ID NOs 1 ou 2, pour utilisation dans le traitement thérapeutique d'un sujet qui nécessite une homéostasie du cholestérol par réduction de la quantité de cholestérol circulant dans le sang du sujet ; ou par augmentation de l'efflux de cholestérol intracellulaire et/ou de la production de HDL dans le foie du sujet.

2. Acide nucléique qui est complémentaire aux SEQ ID NOs 1 ou 2 pour utilisation selon la revendication 1, dans lequel le sujet qui nécessite une homéostasie du cholestérol présente une anomalie cardiovasculaire.

3. Acide nucléique qui est complémentaire aux SEQ ID NOs 1 ou 2 pour utilisation selon la revendication 1 ou 2, dans lequel l'utilisation augmente l'expression de la protéine de cassette de liaison à l'ATP, sous-famille A, membre 1, (ABCA 1) chez le sujet.

4. Acide nucléique qui est complémentaire aux SEQ ID NOs 1 ou 2 pour utilisation selon la revendication 3, lequel acide nucléique présente la SEQ ID N°3.

5. Acide nucléique qui est complémentaire aux SEQ ID NOs 1 ou 2 pour utilisation selon la revendication 3, lequel acide nucléique présente la SEQ ID N°4.

6. Acide nucléique qui est complémentaire aux SEQ ID NOs 1 ou 2 pour utilisation selon l'une quelconque des revendications 1 à 5, qui est un antagomir.

7. Acide nucléique qui est complémentaire aux SEQ ID NOs 1 ou 2 pour utilisation selon l'une quelconque des revendications 1 à 5, qui est un oligonucléotide antisens.

8. Acide nucléique qui est complémentaire aux SEQ ID NOs 1 ou 2 pour utilisation selon l'une quelconque des revendications 1 à 7, qui inclut au moins un nucléotide modifié par un groupe 2'-O-méthyle.

9. Acide nucléique qui est complémentaire aux SEQ ID NOs 1 ou 2 pour utilisation selon l'une quelconque des revendications 1 à 3, qui est un petit ARN interférent (siARN) ou un petit ARN en épingle à cheveux.

10. Acide nucléique qui est complémentaire aux SEQ ID NOs 1 ou 2 pour utilisation selon l'une quelconque des revendications 1 à 9, lequel acide nucléique peut inhiber le traitement post-transcriptionnel de la SEQ ID N° 1.

11. Acide nucléique qui est complémentaire aux SEQ ID NOs 1 ou 2 pour utilisation selon l'une quelconque des revendications 1 à 9, lequel acide nucléique peut inhiber le traitement post-transcriptionnel de la SEQ ID N° 2.
